(19) Europäisches Patentamt
European Patent Office
Office européen des brevets

(11) Veröffentlichungsnummer: **0 223 937 B1**

(12) # EUROPÄISCHE PATENTSCHRIFT

(45) Veröffentlichungstag der Patentschrift: **18.03.92**

(51) Int. Cl.[5]: **C07D 401/14**, C07D 403/04, C07D 413/14, C07D 417/14, C07D 403/14, C07D 409/14, C07D 405/14, C07D 401/04, C07D 413/04, C07D 403/06, A61K 31/395

(21) Anmeldenummer: **86112068.1**

(22) Anmeldetag: **01.09.86**

Die Akte enthält technische Angaben, die nach dem Eingang der Anmeldung eingereicht wurden und die nicht in dieser Patentschrift enthalten sind.

(54) **Heterocyclisch substituierte Indole, Zwischenprodukte, Verfahren zu ihrer Herstellung und Arzneimittel.**

(30) Priorität: **05.09.85 DE 3531658**

(43) Veröffentlichungstag der Anmeldung:
**03.06.87 Patentblatt 87/23**

(45) Bekanntmachung des Hinweises auf die Patenterteilung:
**18.03.92 Patentblatt 92/12**

(84) Benannte Vertragsstaaten:
**AT BE CH DE FR GB IT LI LU NL SE**

(56) Entgegenhaltungen:
**EP-A- 0 052 442**
**EP-A- 0 122 494**
**DE-A- 2 845 220**
**EP-A- 0 085 227**
**EP-A- 0 129 791**
**FR-A- 2 530 246**

(73) Patentinhaber: **BOEHRINGER MANNHEIM GMBH**
**Sandhofer Strasse 116**
**W-6800 Mannheim 31(DE)**

(72) Erfinder: **Mertens, Alfred, Dr. rer. nat.**
**In der Schanz 31**
**W-6905 Schriesheim(DE)**
Erfinder: **Von der Saal, Wolfgang, Dr. rer. nat.**
**Neuer Burgweg 3**
**W-6940 Weinheim(DE)**
Erfinder: **Friebe, Walter-Gunar, Dr. rer. nat.**
**Sopienstrasse 8**
**W-6800 Mannheim 31(DE)**
Erfinder: **Müller-Beckmann, Bernd, Dr. med. vet.**
**Hochgewanne 46**
**W-6718 Grünstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 223 937 B1

JOURNAL OF THE AMERICAN CHEMICAL SOCIETY, Band 106, Nr. 12, 13. Juni 1984, Seiten 3705, 3706, C. WENTRUP et al.: "Synthesis of 1-Azaazulene and Benz[a]azulene by Carbene Rearrangement"

JOURNAL OF THE CHEMICAL SOCIETY (C), 1970, Seiten 829-833; E.B. MULLOCK et al.: "Synthetic uses of polyphosphoric acid and its ethyl ester. Part II. Syntheses of indolin-(3H)-ones and imidazoquinolines"

PATENT ABSTRACTS OF JAPAN, Band 7, Nr. 77 (C-159)[1222], 30. März 1983; & JP - A - 58 8016 (MITSUBISHI KASEI KOGYO K.K.) 18.01.1983

Erfinder: Sponer, Gisbert, Dr. med. vet.
Lessingstrasse 13
W-6941 Laudenbach(DE)

## Beschreibung

Die vorliegende Erfindung betrifft neue Indole der allgemeinen Formel I

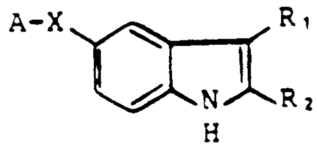

(I),

in welcher

$R_1$ Wasserstoff, die Methyl-, Ethyl-, Isopropyl-, n-Butyl-, Allyl-, Cyclohexyl-, Cyclopentenyl-, Cyan-, Ethoxycarbonyl- oder Phenylgruppe bedeutet,

$R_2$ den Pyrrolyl-, Furanyl-, Thiophenyl-, Pyrazolyl-, Imidazolyl-, Thiazolyl-, Oxazolyl-, Triazolyl-, Tetrazolyl-, Thiadiazolyl-, Oxadiazolyl-, Pyridinyl-, N-Oxy-pyridinyl-, Pyrazinyl-, N,N-Dioxypyrazinyl-, Pyrimidinyl-, N,N-Dioxypyrimidinyl-, Pyridazinyl-, Oxazinyl-, Thiazinyl-, Triazinyl- oder Tetrazinylrest darstellt, sowie deren durch Methyl, Ethyl, Methoxy, Ethoxy, Methylmercapto, Ethylmercapto und Chlorosubstituierten Derivate,
oder den Phenylrest der allgemeinen Formel II

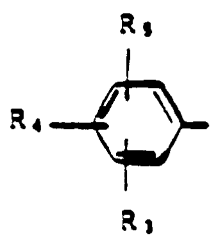

(II)

darstellt, wobei

$R_3$ ein Wasserstoff, die Methylsulfonyloxy-, Trifluormethylsulfonyloxy-, Methylsulfonylamino-, Trifluormethylsulfonylamino-, N-Methyl-methylsulfonylamino-, N-methyl-trifluormethylsulfonylamino, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethyloxy-, Methoxycarbonylmethyloxy-, Cyano-, Chloro-, Nitro-, Amino-, Dimethylamino-, Trifluormethyl- oder die 1-Imidazolylgruppe,

$R_4$ Wasserstoff, die Methyl-, Methoxy-, Dimethylamino- oder Chlorogruppe bedeutet,

$R_5$ Wasserstoff oder die Methoxygruppe ist,

A den 3-Oxo-2,3,4,5-tetrahydro-6-pyridazinyl-, 5-Methyl-3-oxo-2,3,4,5-tetrahydro-6-pyridazinyl-, 5-Hydroxymethyl-3-oxo-2,3,4,5-tetrahydro-6-pyridazinyl-, 3-Cyan-6-methyl-2-oxo-1,2-dihydro-5-pyridinyl-, 3-Aminocarbonyl-6-methyl-2-oxo-1,2-dihydro-5-pyridinyl-, 3-Oxo-2,3,4,5-tetrahydro-1,2,4-triazin-6-yl, 2-Oxo-4,5-dihydro-6H-1,3,4-oxadiazin-5-yl, 6-Oxo-1,4,5,6-tetrahydro-1,2,4-triazin-3-yl-, 5-Oxo-4,5-dihydro-6H-1,3,4-oxadiazin-2-yl-, 2-Oxo-1,2-dihydro-5-pyrimidinyl-, 2-Oxo-1,2-dihydro-5-pyrazinyl-, 4,4-Dimethyl-5-oxo-4,5-dihydro-3-pyrazolyl-, 2-Oxo-4-pyrrolidinyl-, 4-Methyl-5-oxo-4,5-dihydro-1,2,4-triazol-3-yl- oder den 5(4)-Methyl-2-oxo-2,3-dihydro-4(5)-imidazolylrest und

X einen Valenzstrich, die Vinylengruppe oder die Ethylengruppe bedeutet,

deren Tautomere und deren physiologisch verträgliche Salze anorganischer und organischer Säuren und Verfahren zu ihrer Herstellung, sowie diese Verbindungen enthaltende Arzneimittel.

Für den Fall, daß Verbindungen der allgemeinen Formel I hergestellt wurden, die ein asymmetrisches Kohlenstoffatom enthalten, sind auch Gegenstand der Erfindung die optisch aktiven Formen und racemischen Gemische dieser Verbindungen.

Diese neuen Verbindungen der vorliegenden Erfindung weisen wertvolle pharmakologische Eigenschaften auf, insbesondere steigern sie die Herzkraft und/oder wirken blutdrucksenkend und/oder beeinflussen die Thrombozytenfunktion und verbessern die Mikrozirkulation.

Aus der Europäischen Patentanmeldung EP-A-52,442 sind Indolderivate bekannt, die in 5-Stellung durch einen heterocyclischen Sechsring substituiert sind. Diese heterocyclischen Sechsringe sind der 1,3,4-Oxadiazin-2-on-5-yl-, 1,3,4-Thiadiazin-2-on-5-yl-, 1,2,4-Triazin-3-on-6-yl, 1,3,4-Oxadiazin-5-on-2-yl-, 1,3,4-Thiadiazin-5-on-2-yl- und 1,2,4-Thiazin-6-on-3-ylrest. Außerdem können diese Indolderivate am heterocyclischen Fünfring durch Oxo-, Alkyl- oder Alkanoylgruppen substituiert sein. Diese Verbindungen eignen sich zur Behandlung von cardiovaskulären Erkrankungen. Sie wirken insbesondere erhöhend auf die Geschwindigkeit des Druckanstiegs des Blutes in der Aorta und besitzen eine periphere vasodilatierende Wirkung.

Weitere Indolderivate mit ähnlicher Struktur sind bekannt aus DE-A-28 45 220; FR-A-2 530 246; J. Am. Chem. Soc. 106, 3705-6 (1984); J. Chem. Soc. 829 (1970); JP-A-58 8016 (Patent abstracts of Japan, Band 7, Nr. 77); EP-A-85 227 und EP-A-129 791.

Aufgabe der vorliegenden Erfindung gegenüber diesem Stand der Technik war es, neue Indolderivate zur Verfügung zu stellen. Diese Indolderivate sollten zur Herstellung von Arzneimitteln geeignet sein, insbesondere zur Prophylaxe und zur Behandlung von Herz- und Kreislauferkrankungen. Diese Aufgabe wurde dadurch gelöst, daß Indolderivate gefunden wurden, die in 2-Stellung des Indolringes durch eine heterocyclische Gruppe oder durch einen Phenylring der Formel II substituiert sind.

Bedeutet $R_2$ einen Phenylring der allgemeinen Formel II, so kann dieser 1 bis 3 der genannten Substituenten tragen.

Bevorzugte monosubstituierte Phenylverbindungen sind die Hydroxy-, Methyl-, Methoxy-, Propargyloxy-, Cyanmethyloxy-, Methoxycarbonylmethyloxy-, Chlor-, Nitro-, Cyan-, Aminocarbonyl-, Amino-, Dimethylamino-, Methylmercapto-, Methylsulfonyl-, Methylsulfonyloxy- und die 1-Imidazolylverbindungen, wobei der Substituent in 2-, 3- oder 4-Stellung stehen kann.

Bevorzugte disubstituierte Verbindungen enthalten als Substituenten eine Methylsulfonyloxy-, Trifluormethylsulfonyloxy-, Methylsulfenyl-, Methylsulfinylmethyl-, Methylsulfonylmethyl-, Methylsulfonylamino-, N-Methyl-methylsulfonylamino-, Trifluormethylsulfonylamino- oder N-Methyl-trifluormethylsulfonylaminogruppe, eine Aminocarbonyl- oder Aminosulfonylgruppe, eine Methylaminosulfonylgruppe, eine Hydroxy-, Methyl-, Methoxy-, Propargyloxy-, Cyanmethyloxy-, Methoxycarbonylmethyloxy-, Cyan-, Chlor-, Nitro-, Amino-, Dimethylamino-, Methylmercapto-, Methylsulfonyl- oder eine 1-Imidazolylgruppe, wobei die beiden Substituenten gleich oder verschieden sein können und in 2,3-, 2,4-, 2,5-, 2,6-, 3,4- und 3,5-Stellung bevorzugt, jedoch in 2,4-, 2,5- und 3,4-Stellung stehen können.

Bevorzugter trisubstituierter Phenylrest ist der 3,4,5-Trimethoxyphenylrest.

Die Verbindungen der allgemeinen Formel I koennen nach literaturbekannten Verfahren fuer die Indol-Synthese hergestellt werden.

Vergleichehierzu:

(a) P.L. Julian, E.W. Meyer und H.C. Printy, in R.C. Elderfield (Ed.), Heterocyclic Compounds, Vol. 1,S. 1-231, John Wiley and Sons, N.Y. 1952

(b) R.K. Brown, in W.J. Houlihan (Ed.), Heterocyclic Compounds, Vol. 25, Part I, S. 227-537, John Wiley and Sons, N.Y. 1972.

Besonders vorteilhaft ist der in Schema 1 gezeigte Syntheseweg.

Schema 1

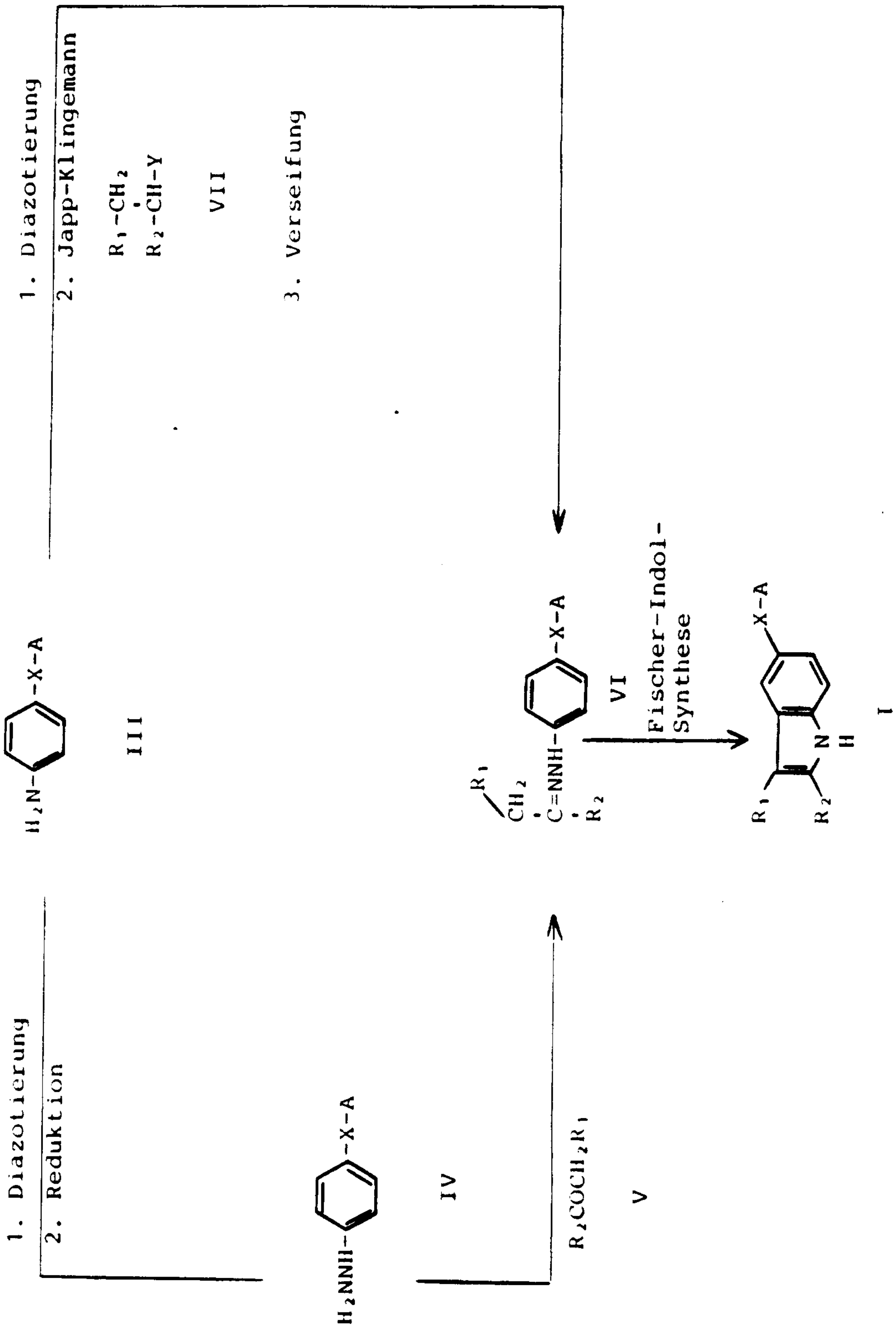


Wie aus Schema 1 ersichtlich lassen sich die literaturbekannten oder nach literaturbekannten Vergleichsverfahren hergestellten Verbindungen der allgemeinen Formel III, in der X und A die angegebene Bedeutung haben, diazotieren, und das Diazoniumsalz zum Hydrazin IV reduzieren. Durch Umsetzung dieser Hydrazine mit Verbindungen der allgemeinen Formel V

$R_2COCH_2R_1$ (V),

in der

$R_1$ und $R_2$ die oben genannte Bedeutung haben, gelangt man zu den Hydrazonen VI, die durch eine Fischer-Indol-Synthese zu Verbindungen der allgemeinen Formel I cyclisiert werden koennen. Andererseits lassen sich die Hydrazone der allgemeinen Formel VI auch dadurch erhalten, daß man das Diazonium-Salz der Amine III in einer Japp-Klingemann-Reaktion mit Verbindungen der allgemeinen Formel VII

$$\begin{array}{l} R_1-CH_2 \\ \quad\;\; | \\ R_2-CH-Y \end{array} \qquad (VII),$$

in der

$R_1$ und $R_2$ die angegebene Bedeutung haben und Y ein die Methingruppe aktivierender Rest ist, umsetzt. Dieser Rest kann z.B. ein Aldehyd, Keton, Ester, Carbonsaeure oder Nitril sein. Die im Reaktionsgemisch intermediaer anfallende Azoverbindung wird ohne Isolierung direkt zum Hydrazon verseift.

Die Verbindungen der Formel VI sind neu und ebenfalls Gegenstand der Erfindung.

Die Diazotierung der Amine III wird vorzugsweise unter neutralen oder sauren Bedingungen in einem polaren Loesungsmittel wie Wasser, Methanol, Ethanol, Eisessig, Salzsaeure, Schwefelsaeure oder Phosphorsaeure bei Temperaturen zwischen -70 °C und 50 °C vorzugsweise jedoch zwischen -5 °C und 10 °C durchgefuehrt.

Zur Diazotierung kommen vorwiegend anorganische Salze oder organische Ester der salpetrigen Saeure in Frage wie z.B. $NaNO_2$, $KNO_2$ oder Amylnitrit.

Die Reduktion der Diazoniumsalze wird ueberwiegend in den oben genannten Loesungsmitteln, in denen die Diazotierung ausgefuehrt wurde, bei Temperaturen zwischen -50 °C und dem Siedepunkt des Loesungsmittels vorgenommen, vorzugsweise jedoch zwischen 0 °C und 80 °C, wobei als Reduktionsmittel Alkalisulfite, Schwefeldioxid, Dithionite, Zinn(II)chlorid, Zinkstaub, Eisen, Natriumamalgam, Triphenylphosphine, Endiole oder auch eine elektrochemische Reduktion in Frage kommen.

Die Umsetzung der Hydrazine mit Verbindungen der allgemeinen Formel V kann in Loesungsmitteln wie Wasser, Alkohol, Benzol, Toluol, Dioxan, DMF, Diethylether oder THF bei Temperaturen zwischen -80 °C bis zum Siedepunkt des verwendeten Loesungsmittel durchgefuehrt werden. Vorteilhaft ist auch der Zusatz eine anorganische oder organische Saeure wie Salzsaeure, Schwefelsaeure, Phosphorsaeure oder Essigsaeure.

Die Japp-Klingemann-Reaktion wird vorteilhaft in den Loesungsmitteln durchgefuehrt, in denen die bereits oben beschriebene Diazotierung durchgefuehrt werden kann. Dies sind also insbesondere Wasser, Methanol, Ethanol, Eisessig, Salzsaeure, Schwefelsaeure oder Phosphorsaeure bei Temperaturen zwischen -50 °C bis 80 °C vorzugsweise jedoch zwischen 0 °C und 25 °C. Die nachfolgende Verseifung kann thermisch oder nach Zusatz eine Base oder Saeure wie z.B. Natronlauge, Kalilauge, Salzsaeure, Schwefelsaeure, Phosphorsaeure oder Eisessig bei Temperaturen bis zum Siedepunkt des Loesungmittels durchgefuehrt werden.

Die Fischer-Indol-Synthese der Hydrazone VI wird ohne Loesungsmittel oder in einem Solvens wie Alkohol, Nitrobenzol, Essigsaeure, Xylol, Cumol, Toluol thermisch oder in Gegenwart eines sauren Katalysator, der jedoch auch Loesungsmittel sein kann, durchgefuehrt, wobei Salzsaeure, Schwefelsaeure, Phosphorsaeure, Polyphosphorsaeure, Eisessig, Ameisensaeure, Zink(II)chlorid, Bortrifluorid, Kationenaustauscher, Sulfosalicylsaeure oder Polyphosphatester in Frage kommen bei Temperaturen zwischen 0 °C und dem Siedepunkt des verwendeten Loesungsmittels.

Die Hydrazone der allgemeinen Formel VI lassen sich gegebenenfalls auch aus den Aminen III ueber die Sydnone X nach Schema 2 herstellen.

Schema 2

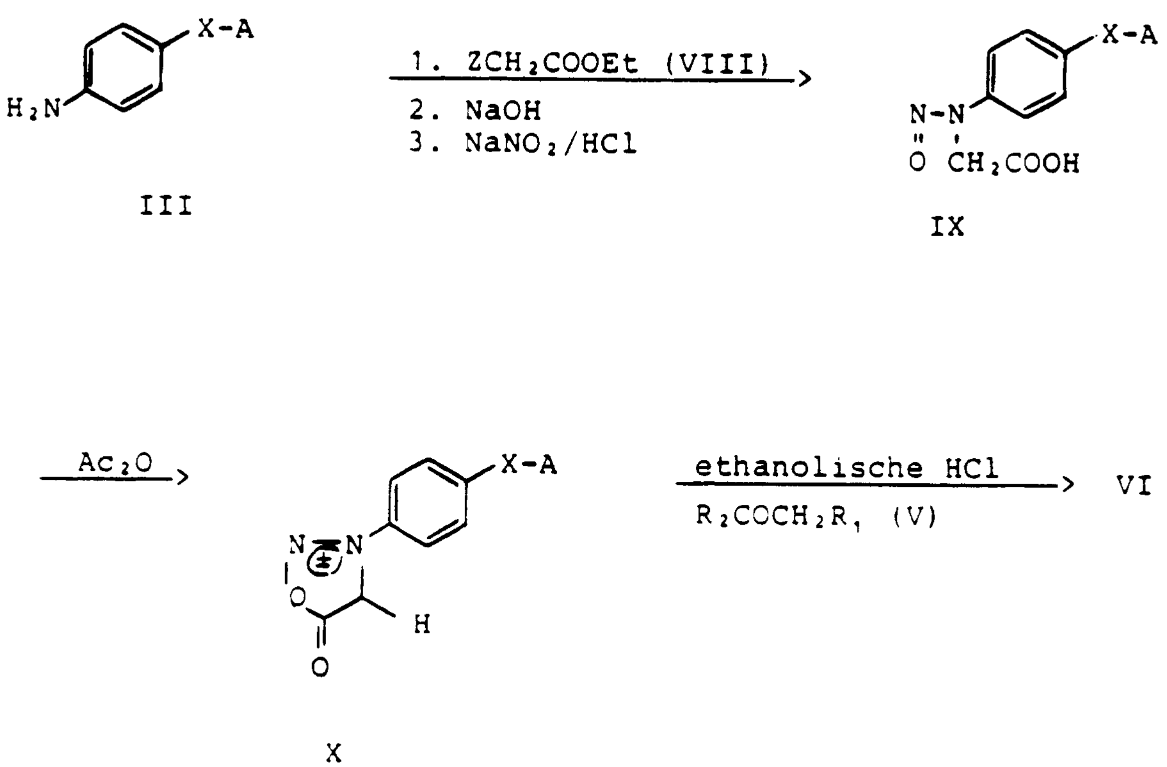


III

IX

X

Die Umsetzung von Aminen III mit Halogenessigestern VIII, in denen Z ein Halogen wie F, Cl, Br oder J, vorzugsweise jedoch Br, bedeutet,
wird vorteilhaft in polaren oder unpolaren Loesungsmittel wie z.B. Methylenchlorid, Toluol, Dioxan, Alkoholen oder Dimethylformamid bei Temperaturen zwischen -50 °C und dem Siedepunkt des Loesungsmittels, vorzugsweise zwischen 25 °C und 100 °C, durchgefuehrt.

Die so erhaltenen Ester koennen nach wohlbekannten Verfahren z.B. mit anorganischen Basen wie z.B. Natriumhydroxid, Kaliumhydroxid, Natriumhydrogencarbonat oder Kaliumhydrogencarbonat in protischen Loesungsmitteln wie Wasser oder Alkohol oder mit anorganischen oder organischen Saeuren wie Salzsaeure, Schwefelsaeure, Eisessig, Phosphorsaeure oder Polyphosphorsaeure gegebenenfalls unter Zugabe eines Loesungsmittels wie Wasser oder Alkohol verseift werden.

Die Nitrosierung der erhaltenen Saeuren zu den Verbindungen der allgemeinen Formel IX wird vorzugsweise unter neutralen oder sauren Bedingungen in einem polaren Loesungsmittel wie Wasser, Methanol, Ethanol, Eisessig, Salzsaeure, Schwefelsaeure oder Phosphorsaeure bei Temperaturen zwischen -70 °C und 50 °C vorzugsweise jedoch zwischen -5 °C und 10 °C durchgefuehrt. Zur Nitrosierung kommen vorwiegend anorganische Salze oder organische Ester der salpetrigen Saeure in Frage wie z.B. $NaNO_2$, $KNO_2$ oder Amylnitrat.

Die Umsetzung der N-Nitroso-carbonsaeuren IX zu den Sydnonen X gelingt in inerten Loesungsmittel wie z.B. Dioxan, Diethylether, Tetrahydrofuran, Toluol mit wasserentziehenden Reagenzien wie z.B. Acetanhydrid, Propionsaeureanhydrid, Schwefelsaeure, Phosphorpentoxid, $PCl_5$ oder $PCl_3$ bei Temperaturen zwischen -50 °C und dem Siedepunkt des Loesungsmittels, vorteilhaft jedoch zwischen 25 °C und 100 °C.

Die Sydnone X lassen sich unter sauren Bedingungen in die Hydrazine IV zerlegen, die in situ mit den Ketonen V zu den Hydrazonen VI abgefangen werden. Als Saeuren fuer die Verseifung der Sydnone kommen Salzsaeure, Schwefelsaeure, Phosphorsaeure, Polyphorphorsaeure oder organische Saeuren wie Eisessig bei Temperaturen zwischen -70 °C und 100 °C vorzugsweise zwischen 0 °C und 70 °C in Betracht.

Verbindungen der allgemeinen Formel I können auch nachträglich in eine andere Verbindung der Formel I umgewandelt werden. Dies trifft zum Beispiel zu

a) für die Oxidation eines Fünf- oder Sechsringes mit einem oder mehreren Stickstoffatomen zu den entsprechenden N-Oxiden. Die Oxidation wird zweckmäßig mit einem oder mehreren Äquivalenten des

verwendeten Oxidationsmittels durchgeführt, z. B. mit Wasserstoffperoxid in Eisessig, Trifluoressigsäure oder in Ameisensäure bei 20-100 °C oder in Aceton bei 0-60 °C, mit einer Persäure wie Perameisensäure oder m-Chlorperbenzoesäure in Eisessig, Trifluoressigsäure, Methylenchlorid oder Chloroform bei Temperaturen zwischen 0 und 60 °C.

b) für die Herstellung von Verbindungen der allgemeinen Formel I, in der $R_2$ einen Rest der allgemeinen Formel II bedeutet und $R_3$ eine Methylsulfonyl-, Methylsulfinylmethyl- oder Methylsulfonylmethylgruppe darstellt, durch nachträgliche Oxidation einer Verbindung der allgemeinen Formel XI,

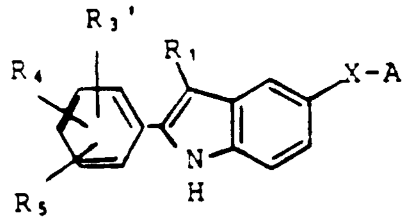

(XI)

in der
$R_1$, $R_4$, $R_5$, A und X wie eingangs definiert sind und $R_3$' eine Methylmercapto- oder Methylsulfenylmethylgruppe ist. Die Oxidation wird vorzugsweise in einem Lösungsmittel oder Lösungsmittelgemisch, z. B. in Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80° und 100 °C durchgeführt.

Zur Herstellung einer Methylsulfinylmethylverbindung der allgemeinen Formel I wird die Oxidation zweckmäßigerweise mit einem Äquivalent des verwendeten Oxidationsmittel durchgeführt, z. B. mit Wassersoffperoxid in Eisessig, Trifluoressigsäure oder Ameisensäure bei 0 bis 20 °C oder in Aceton bei 0 bis 60 °C, mit einer Persäure wie Perameisensäure in Eisessig oder Trifluoressigsäure bei 0 bis 50 °C oder mit m-Chlorperbenoesäure in Methylenchlorid oder Chloroform bei -20 bis 60 °C, mit Natriummetaperjodat in wässrigem Methanol oder Ethanol bei -15 bis 25 °C, mit Brom in Eisessig oder wässriger Essigsäure, mit N-Brom-succinimid in Ethanol, mit tert.-Butyl-hypochlorid in Methanol bei -80 bis -30 °C, mit Jodbenzodichlorid in wässrigem Pyridin bei 0 bis 50 °C, mit Salpetersäure in Eisessig bei 0 bis 20 °C, mit Chromsäure in Eisessig oder in Aceton bei o bis 20 °C und mit Sulfurylchlorid in Methylenchlorid bei -70 °C, der hierbei erhaltenen Thioether-Chlor-Komplex wird zweckmäßigerweise mit wässrigem Ethanol hydrolysiert.

Zur Herstellung einer Methylsulfonyl- oder Methylsulfonylmethylverbindung der allgemeinen Formel I wird die Oxidation zweckmäßigerweise mit einem bzw. mit zwei oder mehr Äquivalenten des verwendeten Oxidationsmittels durchgeführt, z. B. Wasserstoffperoxid in Eisessig, Trifluoressigsäure oder in Ameisensäure bei 20 bis 100 °C oder in Aceton bei 0 bis 60 °C, mit einer Persäure wie Perameisensäure oder m-Chlorperbenzoesäure in Eisessig, Trifluoressigsäure, Methylenchlorid oder Chloroform bei Temperaturen zwischen 0 und 60 °C, mit Salpetersäure in Eisessig bei 0 bis 20 °C, mit Chromsäure oder Kaliumpermanganat in Eisessig, Wasser/Schwefelsäure oder in Aceton bei 0 bis 20 °C.

c) für die Herstellung von Verbindungen der allgemeinen Formel I, in der $R_2$ einen Rest der allgemeinen Formel II bedeutet und $R_3$ eine Methylsulfonyloxy-, Trifluormethansulfonyloxy-, Methylsulfonylamino- , M-Methyl-methansulfonylamino-, Trifluormethansulfonylamino- oder N-Methyl-trifluormethansulfonylaminogruppe darstellt, durch die nachträgliche Umsetzung einer Verbindung der allgemeinen Formeln XII

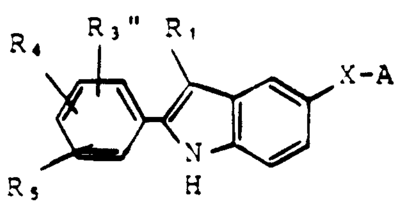

(XII),

in der
$R_1$, $R_4$, $R_5$, A und X eingangs definiert sind und $R_3$'' eine Hydroxy-, Amino- oder N-Methylaminogruppe darstellt, mit einer Sulfonsäure der allgemeinen Formel XIII

$R_6$-$SO_2$-OH (XIII),

in der

$R_6$ eine Methylgruppe oder eine Trifluormethylgruppe darstellt, in Gegenwart eines wasserentziehenden und/oder die Säure oder das Amin aktivierenden Mittels oder mit deren reaktionsfähigen Derivaten.

Die Umsetzung wird zweckmäßigerweise in einem Lösungsmittel oder Lösungsmittelgemisch wie Methylenchlorid, Ether, Tetrahydrofuran, Dioxan oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der allgemeinen Formel XIII z. B. mit deren Anhydrid oder Halogenid wie Methansulfonsäurechlorid oder Ethansulfonsäurechlorid, vorzugsweise bei Temperaturen zwischen 0 und 100 ° C, z. B. bei Temperaturen zwischen Raumtemperatur und 50 ° C, durchgeführt.

d) für die Herstellung von Verbindungen der allgemeinen Formel I, in der $R_2$ einen Rest der allgemeinen Formel II bedeutet und $R_3$ eine Aminocarbonyl- oder Aminosulfonylgruppe darstellt durch die nachträgliche Umsetzung einer Verbindung der allgemeinen Formel XIV

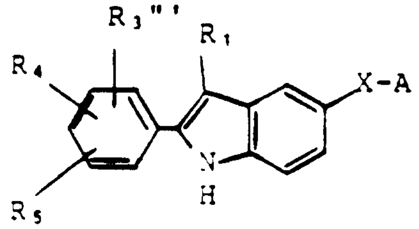

(XIV) ,

in der

$R_1$, $R_4$, $R_5$, A und X wie eingangs definiert sind und $R_3'''$ eine Carboxyl- oder Hydroxysulfonylgruppe darstellen, oder einem reaktionsfähigen Derivat hiervon wie z. B. Ester oder Säurechlorid mit Hydrazin oder einem Amin der allgemeinen Formel XV

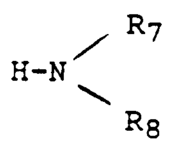

(XV) ,

in der

$R_7$ und $R_8$, die gleich oder verschieden sein koennen, Wasserstoffatome oder Alkylgruppen mit 1-5 Kohlenstoffatomen darstellen, oder mit einem reaktionsfaehigen Derivat hiervon, falls $R_3'''$ die Carboxyl- oder Hydroxysulfonylgruppe darstellen.

Die Umsetzung wird zweckmaeßigerweise in einem Loesungsmittel wie Methylenchlorid, Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Saeure aktivierenden Mittels oder eines wasserentziehenden Mittels, z.B. in Gegenwart von Chlorameisensaeureethylester, Thionylchlorid, Phosphortrichlorid, Phosphorpentoxid, N-N ±Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Hydrazino- oder Aminogruppe aktivierenden Mittels, z.B. Phosphortrichlorid, und gegebenenfalls in Gegenwart einer anorganischen Base wie Natriumcarbonat oder einer tertiaeren organischen Base wie Triethylamin oder Pyridin, welche gleichzeitig als Loesungsmittel dienen koennen, bei Temperaturen zwischen -25 und 250 ° C, vorzugsweise jedoch bei Temperaturen zwischen -10 ° C und der Siedetemperatur des verwendeten Loesungsmittels durchgefuehrt werden, desweiteren kann waehrend der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Besonders vorteilhaft wird jedoch die Umsetzung mit einem entsprechenden Halogenid, z.B. dem Carbonsaeure- oder Sulfonsaeurechlorid, und Hydrazin oder einem entsprechenden Amin, wobei diese gleichzeitig als Loesungsmittel dienen koennen, und bei Temperaturen zwischen 0 und 50 ° C durchgefuehrt.

e) für die Herstellung von Verbindungen der allgemeinen Formel I, in der $R_1$ eine Cyangruppe bedeutet, durch nachträgliche Umsetzung einer Verbindung der allgemeinen Formel I, in der $R_2$, A und X die angegebene Bedeutung haben, und $R_1$ Wasserstoff bedeutet, mit N-Carbonylsulfamoylchlorid, das auch als Chlorsulfonylisocyanat bezeichnet wird, in einem geeigneten Lösungsmittel nach an sich bekannten Verfahren (Chem. Ber. 100, 2719 (1967); Synthesis 1978, 374 und J. Chem. Soc., Perkin I, 1978, 1117).
Die Reaktion wird zweckmäßig in einem unter den Reaktionsbedingungen inerten Lösungsmittel, z. B. Wasser, Methanol, Ethanol, n-Butanol, Dioxan, Acetonitril, Nitromethan, Pyridin, Dimethylformamid oder Methylenchlorid, gegebenenfalls in Gegenwart eines säurebindenden Mittels durchgeführt.
Die Reaktion wird unter Eiskühlung, bei Raumtemperatur oder unter Erwärmen, gegebenenfalls unter einer Schutzgasatomosphäre, ausgeführt.
f) für die Herstellung von Verbindungen der allgemeinen Formel I in der $R_1$ die Ethoxycarbonylgruppe bedeutet, oder $R_2$ ein Rest der allgemeinen Formel II ist, wobei $R_3$ eine Aminocarbonyl- oder Methoxycarbonylmethoxygruppe bedeutet, durch nachträgliche Alkoholyse und/oder Hydrolyse von Verbindungen der allgemeinen Formel I, in der $R_1$ und $R_2$ die Cyangruppe bedeutet oder $R_2$ ein Rest der allgemeinen Formel II ist, wobei $R_3$ eine Cyan- oder Cyanalkyloxygruppe darstellt.
Die nachträgliche Alkoholyse und/oder Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Ethanol, Wasser/Ethanol, Wasser/Isopropanol und Wasser/Dioxan bei Temperaturen zwischen -10 und 120°C, z.B., bei Temperaturen zwischen Raumtemperatur und der Siedetemperatur des Reaktionsgemisches, durchgefuehrt.
Ferner koennen die erhaltenen Verbindungen der allgemeinen Formel I anschließend gewuenschtenfalls in ihre physiologisch vertraeglichen Saeureadditionssalze mit anorganischen oder organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.
Wie bereits eingangs erwaehnt weisen die neuen Verbindungen der allgemeinen Formel I, deren Tautomere und deren physiologisch vertraegliche Saeureadditionssalze bei einer langen Wirkungsdauer ueberlegene pharmakologische Eigenschaften auf, insbesondere eine blutdrucksenkende und/oder positivinotrope Wirkung und/oder beeinflussen die Thrombozytenfunktion und verbessern die Mikrozirkulation.
Zur Herstellung von Arzneimitteln werden die Substanzen der allgemeinen Formel I in an sich bekannter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.
Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.
Derartige Zusaetze sind z.B. Tartrat- und Citratpuffer, Ethanol,Komplexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Stearinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.
Die erfindungsgemaeßen Verbindungen werden ueblicherweise in Mengen von 10-500 mg pro Tage bezogen auf 75 kg Koerpergewicht appliziert. Bevorzugt ist es, 2-3 mal pro Tag 1-2 Tabletten mit einem Wirkstoffgehalt von 5-200 mg zu verabreichen. Die Tabletten koennen retardiert sein, wodurch nur noch 1 mal pro Tag 1-2 Tabletten mit 10-500 mg Wirkstoff gegeben werden muß. Der Wirkstoff kann auch durch Injektion 1-8 mal pro Tag bzw. durch Dauerinfusion gegeben werden, wobei Mengen von 5-200 mg/Tag normalerweise ausreichen.

Bevorzugt im Sinne der vorliegenden Erfindung sind außer den in den Beispielen genannten Verbindungen die folgenden und deren Tautomere:

2-(2-Pyridyl)-5-(3-oxo-2,3,4,5-tetrahydro-6-pyridazinyl)-indol 2-(5-Methylmercapto-1,3,4-oxadiazol-2-yl)-5-(3-oxo-2,3,4,5-tetrahydro-6-pyridazinyl)-indol
2-(1,2,5-Thiadiazol-3-yl)-5-(3-oxo-2,3,4,5-tetrahydro-6-pyridazinyl)-indol
2-(N-Oxy-4-pyridyl)-5-(3-oxo-2,3,4,5-tetrahydro-6-pyridazinyl)-indol
2-(4-Nitro-phenyl)-5-(3-oxo-2,3,4,5-tetrahydro-6-pyridazinyl)-indol

2-(4-Amino-phenyl)-5-(3-oxo-2,3,4,5-tetrahydro-6-pyridazinyl)-indol
2-Methyl-5-(3-oxo-2,3,4,5-tetrahydro-6-pyridazinyl)indol
2-Trifluormethyl-5-(3-oxo-2,3,4,5-tetrahydro-6-pyridazinyl)-indol
2-Cyclopropyl-5-(3-oxo-2,3,4,5-tetrahydro-6-pyridazinyl)indol
2-Cyano-5-(3-oxo-2,3,4,5-tetrahydro-6-pyridazinyl)indol
2-(5-Pyrimidinyl)-5-(3-oxo-2,3,4,5-tetrahydro-6-pyridazinyl)-indol
2-(4-Imidazolyl)-5-(3-oxo-2,3,4,5-tetrahydro-6-pyridazinyl)-indol
2-(1,2,4-Triazol-3-yl)-5-(3-oxo-2,3,4,5-tetrahydro-6-pyridazinyl)indol
2-(2-Methoxy-4-methylsulfonyloxy-phenyl)-5-(3-oxo-2,3,4,5-tetrahydro-6-pyridazinyl)indol
2-(2-Methoxy-4-propargyloxy-phenl)-5-(3-oxo-2,3,4,5-tetrahydro-6-pyridazinyl)indol
2-(2-Methoxy-4-cyanomethyloxy-phenyl)-5-(3-oxo-2,3,4,5-tetrahydro-6-pyridazinyl)indol
2-(2-Methoxy-4-allyloxy-phenyl)-5-(3-oxo-2,3,4,5-tetrahydro-6-pyridazinyl)indol
2-(2-Methoxy-4-carboxymethyloxy-phenyl)-5-(3-oxo-2,3,4,5-tetrahydro-6-pyridazinyl)indol
5-(3-Oxo-2,3,4,5-tetrahydro-6-pyridazinyl)indol
2-(4-Methylsulfenyl-2-methoxy-phenyl)-5-(5-methyl-3-oxo-2,3,4, 5-tetrahydro-6-pyridazinyl)indol
2-(4-Methylsulfinyl-2-methoxy-phenyl)-5-(5-methyl-3-oxo-2,3,4, 5-tetrahydro-6-pyridazinyl)indol
2-(4-Methylsulfonyl-2-methoxy-phenyl)-5-(5-methyl-3-oxo-2,3,4, 5-tetrahydro-6-pyridazinyl)indol
3-Methyl-5-(5-methyl-3-oxo-2,3,4,5-tetrahydro-6-pyridazinyl)-indol
2,3-Dimethyl-5-(5-methyl-3-oxo-2,3,4,5-tetrahydro-6-pyridazinyl)indol
2-Methyl-3-cyan-5-(5-methyl-3-oxo-2,3,4,5-tetrahydro-6-pyridazinyl)indol
2-Methyl-3-aminocarbonyl-5-(5-methyl-3-oxo-2,3,4,5-tetrahydro-6-pyridazinyl)indol
2-Methyl-3-ethoxycarbonyl-5-(5-methyl-3-oxo-2,3,4,5-tetrahydro-6-pyridazinyl)indol
2-(Cyclohexen-1-yl)-5-(5-methyl-3-oxo-2,3,4,5-tetrahydro-6-pyridazinyl)indol
2-Acetyl-5-(5-methyl-3-oxo-2,3,4,5-tetrahydro-6-pyridazinyl)-indol
2,3-Diphenyl-5-(5-methyl-3-oxo-2,3,4,5-tetrahydro-6-pyridazinyl)indol
2-(3,4,5-Trimethoxy-phenyl)-5-(5-methyl-3-oxo-2,3,4,5-tetrahydro-6-pyridazinyl)indol
2-(2-Hydroxy-5-pyrimidinyl)-5-(5-methyl-3-oxo-2,3,4,5-tetrahydro-6-pyridazinyl)indol
2-(4-Pyrimidinyl)-5-(5-methyl-3-oxo-2,3,4,5,-tetrahydro-6-pyridazinyl)indol
2-(4-Pyridyl-5-(5-hydroxymethyl-3-oxo-2,3,4,5-tetrahydro-6-pyridazinyl)indol
2-(2-Methyl-4-oxazolyl)-5-(5-hydroxymethyl-3-oxo-2,3,4,5-tetrahydro-6-pyridazinyl)-indol
2-(2-Chlor-4-pyridyl)-5-(5-hydroxymethyl-3-oxo-2,3,4,5-tetrahydro-6-pyridazinyl)-indol
2-(2-Methyl-4-pyridyl-5-(5-hydroxymethyl-3-oxo-2,3,4,5-tetrahydro-6-pyridazinyl)-indol
2-(4-Trifluormethylsulfonyl-2-methoxy-phenyl-5-(5-hydroxymethyl-3-oxo-2,3,4,5-tetrahydro-6-pyridazinyl)-indol
2-(4-Morpholinylsulfonyl-2-methoxy-phenyl)-5-(5-hydroxymethyl-3-oxo-2,3,4,5-tetrahydro-6-pyridazinyl)-indol
2-(2-Furanyl)-5-(5-hydroxymethyl-3-oxo-2,3,4,5-tetrahydro-6-pyridazinyl)-indol
2-(2,4-Dimethoxy-phenyl)-5-(5-hydroxymethyl-3-oxo-2,3,4,5-tetrahydro-6-pyridazinyl)-indol
2-(4-Aminosulfonyl-2-methoxy-phenyl)-5-(5-hydroxymethyl-3-oxo-2,3,4,5-tetrahydro-6-pyridazinyl)-indol
2-Methyl-5-(3-cyan-6-methyl-2-oxo-1,2-dihydro-5-pyridyl)-indol
2-Phenyl-5-(3-cyan-6-methyl-2-oxo-1,2-dihydro-5-pyridyl)-indol
2-(4-Pyridazinyl)-5-(3-cyan-6-methyl-2-oxo-1,2-dihydro-5-pyridyl)-indol
2-(5-Pyrimidinyl)-5-(3-cyan-6-methyl-2-oxo-1,2-dihydro-5-pyridyl)-indol
2-(4-Pyridyl)-5-(3-aminocarbonyl-6-methyl-2-oxo-1,2-dihydro-5-pyridyl)-indol, Schmp. 156-157 °C.
2-(2-Pyrazinyl)-5-(3-oxo-2,3,4,5-tetrahydro-1,2,4-triazin-6-yl)-indol
2-(2-Pyrrolyl)-5-(3-oxo-2,3,4,5-tetrahydro-1,2,4-triazin-6-yl)-indol
2-(1,2,4-Triazol-3-yl)-5-(3-oxo-2,3,4,5-tetrahydro-1,2,4-triazin-6-yl)-indol
2-(2-Thienyl)-5-(3-oxo-2,3,4,5-tetrahydro-1,2,4-triazin-6-yl)-indol
2-Methyl-5-(2-oxo-2,3-dihydro-6H-1,3,4-oxadiazin-5-yl)-indol
2-(4-Pyridazinyl)-5-(2-oxo-2,3-dihydro-6H-1,3,4-oxadiazin-5-yl)-indol
2-(2-Furanyl)-5-(2-oxo-2,3-dihydro-6H-1,3,4-oxadiazin-5-yl)-indol
2-Phenyl-5-(2-oxo-2,3-dihydro-6H-1,3,4-oxadiazin-5-yl)-indol
2-(5-Pyrimidinyl)-5-(6-oxo-1,4,5,6-tetrahydro-1,2,4-triazin-3-yl)-indol
2-(2-Pyrrolyl)-5-(6-oxo-1,4,5,6-tetrahydro-1,2,4-triazin-3-yl)-indol
2-(4-Pyridyl)-5-(6-oxo-1,4,5,6-tetrahydro-1,2,4-triazin-3-yl)-indol
2-(1,2,4-Triazol-3-yl)-5-(6-oxo-1,4,5,6-tetrahydro-1,2,4-triazin-3-yl)-indol
2-Methyl-5-(5-oxo-4,5-dihydro-6H-1,3,4-oxadiazin-2-yl)-indol
2-Phenyl-5-(5-oxo-4,5-dihydro-6H-1,3,4-oxadiazin-2-yl)-indol
2-(2-Pyrazinyl)-5-(5-oxo-4,5-dihydro-6H-1,3,4-oxadiazin-2-yl)-indol

2-(2-Thienyl)-5-(5-oxo-4,5-dihydro-6H-1,3,4-oxadiazin-2-yl)-indol
2-(2-Furanyl)-5-(5-oxo-4,5-dihydro-6H-1,34-oxadiazin-2-yl)-indol
2-(3-Pyridyl)-5-(2-oxo-1,2-dihydro-5-pyrimidinyl)-indol
2-(4-Pyridazinyl)-5-(2-oxo-1,2-dihydro-5-pyrimidinyl)-indol
2-Phenyl-5-(2-oxo-1,2-dihydro-5-pyrimidinyl)-indol
2-Trifluormethyl-5-(2-oxo-1,2-dihydro-5-pyrimidinyl)-indol
2-(4-Pyridyl)-5-(4-methyl-2-oxo-1,2-dihydro-5-pyrimidinyl) indol
2-Acetyl-5-(2-oxo-1,2-dihydro-5-pyrazinyl)-indol
2-(5-Pyrimidinyl)-5-(2-oxo-1,2-dihydro-5-pyrazinyl)-indol
2-(2-Pyrazinyl)-5-(2-oxo-1,2-dihydro-5-pyrazinyl)-indol
2-(2-Pyrrolyl)-5-(2-oxo-1,2-dihydro-5-pyrazinyl)-indol
2-(4-Thiazolyl)-5-(2-oxo-1,2-dihydro-5-pyrazinyl)-indol
2-Methyl-5-(4,4-dimethyl-5-oxo-4,5-dihydro-3-pyrazolyl)-indol
2-Phenyl-5-(4,4-dimethyl-5-oxo-4,5-dihydro-3-pyrazolyl)-indol
2-(4-Pyridazinyl)-5-(4,4-dimethyl-5-oxo-4,5-dihydro-3-pyrazolyl)-indol
2-(2-Furanyl)-5-(4,4-dimethyl-5-oxo-4,5-dihydro-3-pyrazolyl)-indol
2-(4-Thiazolyl)-5-(4,4-dimethyl-5-oxo-4,5-dihydro-3-pyrazolyl)-indol
2-(4-Pyridyl)-5-(2-oxo-4-pyrrolidinyl)-indol
2-Phenyl-5-(2-oxo-4-pyrrolidinyl)-indol
2-(4-Pyridazinyl)-5-(2-oxo-4-pyrrolidinyl)-indol
2-(2-Thienyl)-5-(2-oxo-4-pyrrolidinyl)-indol
2-(1,2,4-Triazol-3-yl)-5-(2-oxo-4-pyrrolidinyl)-indol
2-(3-Pyridyl)-5-(4-methyl-5-oxo-4,5-dihydro-1,2,4-triazol-3-yl)-indol
2-(2-Pyridyl)-5-(4-methyl-5-oxo-4,5-dihydro-1,2,4-triazol-3-yl)-indol
2-(N-Oxy-4-pyridyl)-5-(4-methyl-5-oxo-4,5-dihydro-1,2,4-triazol-3-yl)-indol
2-Ethoxycarbonyl-5-(4-methyl-5-oxo-4,5,-dihydro-1,2,4-triazol-3-yl)-indol
2-Trifluormethyl-5-(4-methyl-5-oxo-4,5-dihydro-1,2,4-triazol-3-yl)-indol
2-(4-Pyridyl)-5-[4(5)-methyl-2-oxo-2,3-dihydro-5(4)-imadazolyl]-indol
2-(4-Pyridazinyl)-5-[4(5)-methyl-2-oxo-2,3-dihydro-5(4)-imidazolyl]-indol
2-(4-Pyridyl)-5-[2-oxo-2,3-dihydro-4(5)-imidazolyl)-indol
2-Methyl-5-[2-oxo-2,3-dihydro-4(5)-imadazolyl]-indol
2-Cyclopropyl-5-[2-oxo-2,3-dihydro-4(5)-imidazolyl]-indol
2-(4-Pyridyl)-5-[2-(3-oxo-2,3,4,5-tetrahydro-6-pyridazinyl)-ethyl]-indol
2-(4-Pyridazinyl)-5-[2-(3-oxo-2,3,4,5-tetrahydro-6-pyridazinyl)-ethyl]-indol
2-Phenyl-5-[2-(3-oxo-2,3,4,5-tetrahydro-6-pyridazinyl)ethyl]-indol
2-(4-Pyridyl)-5-[2-(3-oxo-2,3,4,5-tetrahydro-6-pyridazinyl)-vinyl]-indol
2-Methyl-5-[2-(3-oxo-2,3,4,5-tetrahydro-6-pyridazinyl)vinyl]-indol
2-(4-Pyridazinyl)-5-[2-(3-oxo-2,3,4,5-tetrahydro-6-pyridazinyl)-vinyl]-indol

## Beispiel 1

2-(4-Pyridyl)-5-(3-oxo-2,3,4,5-tetrahydro-6-pyridazinyl)-indol

4.0 g (21.1 mmol) 6-(4-Amino-phenyl)-2,3,4,5-tetrahydro-pyridazin-3-on wurden mit 70 ml 50proz. Schwefelsaeure versetzt und auf 0°C gekuehlt. In die Suspension wurden bei 0°C 1.53 g (22.2 mmol) Natriumnitrit, in 12 ml Wasser geloest, zugetropft. Nach beendeter Zugabe wurde 15 min nachgeruehrt und 0.26 g (4.3 mmol) Harnstoff zugesetzt und weitere 15 min geruehrt. Anschließend wurde bei 0°C eine Loesung von 14.35 g (63.6 mmol) Zinn(II)-chlorid Dihydrat in 12 ml konz. Salzsaeure zugetropft und weitere 2 h bei 0°C nachgeruehrt. Unter Kuehlung wurden nun 3.8 g (31.4 mmol) 4-Acetylpyridin zugetropft und 2 h bei 25°C weitergeruehrt. Nach Stehen ueber Nacht wurde der Rueckstand abgesaugt, mit Wasser gewaschen, erneut in Wasser suspendiert, mit Ammoniak auf pH 8.5 gestellt, abgesaugt und getrocknet. Man erhaelt als Rohprodukt 6.76 g 4-Acetylpyridin-[4-(3-oxo-2,3,4,5-tetrahydro-6-pyridazinyl)phenylhydrazon], welches ohne weitere Reinigung umgesetzt wurde.

6 g (19.5 mmol) des Hydrazons wurden unter Stickstoff in 40 ml Polyphosphorsaeure fuer 2 h bei 120°C geruehrt. Anschließend wurde auf Eis gegossen, mit konz. Ammoniak neutralisiert und abgesaugt. Der Rueckstand wurde mit 1500 ml einer 1:1 Mischung Methylenchlorid/Methanol warm ausgeruehrt, das Filtrat gekohlt, eingeengt, abgesaugt und durch Saeulenchromatographie (Laufmittel: Methylenchlorid/Methanol 8:2) gereinigt.

Ausbeute: 0.81 g (14.3 %), Schmp. 314-16 ° C.

Beispiel 2

2-(4-Pyridyl)-3-ethyl-5-(3-oxo-2,3,4,5-tetrahydro-6-pyridazinyl)-indol

a) 9.45 g (49.9 mmol) 6-(4-Aminophenyl)-2,3,4,5-tetrahydropyridazin-3-on wurden in 100 ml 2 N Salzsaeure eingetragen und auf - 2 bis 0 ° C gekuehlt. Bei dieser Temperatur wurden 3.5 g (50.7 mmol) Natriumnitrit, geloest in 10 ml Wasser, zugetropft und 15 min nachgeruehrt. Anschließend wurden 28 g (124 mmol) Zinn (II) chlorid Dihydrat, geloest in 20.5 ml konz. Salzsaeure, zugetropft und 60 min bei 0 ° C weitergeruehrt. Der Niederschlag wurde kalt abgesaugt und mit wenig 2 N Salzsaeure und Wasser nachgewaschen. Man erhaelt 8.7 g 6-(4-Hydrazinophenyl)-2,3,4, 5-tetrahydro-pyridazin-3-on als Hydrochlorid vom Schmp. 243 ° C Zers.

b) 4 g (16.6 mmol) des unter a) erhaltenen Hydrazins wurden in 50 ml Ethanol und 50 ml Wasser mit 2.97 g (19.9 mmol) 4-Butyryl-pyridin versetzt und 3 h bei 25 ° C weitergeruehrt. Der ausgefallene Niederschlag wurde abgesaugt, mit Ethanol/Wasser 1:1 nachgewaschen, nochmals in Wasser suspendiert, mit waessrigem Ammoniak neutralisiert und abgesaugt. Man erhaelt 2.9 g 4-Butyryl-pyridin-[4-(3-oxo-2,3,4,5-tetrahydro-6-pyridazinyl) phenylhydrazon] vom Schmp. 243-46 ° C.

c) 2.7 g (8 mmol) des nach b) erhaltenen Hydrazons wurden in 20 ml Polyphosphorsaeure 3 h unter Stickstoff auf 120 ° C erhitzt. Die noch warme Loesung wurde aus Eis/Wasser gegossen, durchgearbeitet und abgesaugt. Der Rueckstand wurde nochmals in Wasser suspendiert, mit waessrigem Ammoniak neutralisiert, abgesaugt und aus Methanol unter Zusatz von Kohle umkristallisiert. Man erhaelt 1.65 g (64,5 %) der Titelverbindung vom Schmp. > 300 ° C.

Beispiel 3

2-(4-Pyridyl)-3-isopropyl-5-(3-oxo-2,3,4,5-tetrahydro-6-pyridazinyl)indol

Analog Beispiel 2 erhaelt man aus 6-(4-Hydrazinophenyl)-2,3,4,5-tetrahydro-pyridazin-3-on und 4-(3-Methyl-butyryl) pyridin 57 % des 4-(3-Methyl-butyryl)pyridin-[4-(3-oxo-2,3,4,5-tetrahydro-6-pyridazinyl)-phenylhydrazon] vom Schmp. 225 ° C Zers., das in Polyphosphorsaeure zur Titelverbindung cyclisiert wurde.
Ausbeute: 41 %, Schmp. > 300 ° C aus Methanol.

Beispiel 4

2-(4-Aminocarbonyl-phenyl)-5-(3-oxo-2,3,4,5-tetrahydro-6-pyridazinyl)indol

Analog Beispiel 2 erhaelt man aus 6-(4-Hydrazinophenyl)-2,3,4,5-tetrahydro-pyridazin-3-on und p-Cyanoacetophenon 96 % des p-Cyanoacetophenon-[4-(3-oxo-2,3,4,5-tetrahydro-6-pyridazinyl) phenylhydrazon] vom Schmp. 288-90 ° C (Ethanol), welches in Polyphosphorsaeure zur Titelverbindung cyclisiert wurde.
Ausbeute: 56 %, Schmp. > 300 ° C.

Beispiel 5

2-(3-Thienyl)-5-(3-oxo-2,3,4,5-tetrahydro-6-pyridazinyl) indol

Analog Beispiel 2 erhaelt man aus 6-(4-Hydrazinophenyl)-2,3,4,5-tetrahydro-pyridazin-3-on und 3-Acetylthiophen das 3-Acetylthiophen-[4-(3-oxo-2,3,4,5-tetrahdyro-6-pyridazinyl)phenylhydrazon] als Rohprodukt, welches in Polyphosphorsaeure zur Titelverbindung cyclisiert und durch Saeulenchromatographie (Laufmittel: Methylenchlorid / mit Ammoniak gesaettigtes Methanol 20:1 ) gereinigt wurde.
Ausbeute: 17 %, Schmp. 325-33 ° C.

Beispiel 6

2-(2-Chlorphenyl)-5-(3-oxo-2,3,4,5-tetrahydro-6-pyridazinyl)indol

Analog Beispiel 2 erhaelt man aus 6-(4-Hydrazinophenyl)-2,3,4,5-tetrahydro-pyridazin-3-on und 2-Chloracetophenon das 2-Chloracetophenon-[4-(3-oxo-2,3,4,5-tetrahydro-6-pyridazinyl) phenylhydrazon] als Rohprodukt, welches in Polyphosphorsaeure zur Titelverbindung cyclisiert wurde.
Ausbeute: 39 %, Schmp. 236-39 °C aus Ethanol/Wasser 10:1.

Beispiel 7

2-(3-Trifluormethyl-phenyl)-5-(3-oxo-2,3,4,5-tetrahydro-6-pyridazinyl)indol

Analog Beispiel 2 erhaelt man aus 6-(4-Hydrazinopheny)-2,3,4,5-tetrahydro-pyridazin-3-on und 3-Trifluormethyl-acetophenon das 3-Trifluormethyl-acetophenon-[4-(3-oxo-2,3,4,5-tetrahydro-6-pyridazinyl)-phenylhydrazon] als Rohprodukt, welches in Polyphosphorsaeure zur Titelverbindung cyclisiert und durch Saeulenchromatographie (Laufmittel: Methylenchlorid / mit Ammoniak gesaettigtes Methanol 20:1) gereinigt wurde.
Ausbeute: 30 %, Schmp. 215-18 °C aus Methanol

Beispiel 8

2-(4-Pyridyl)-3-methyl-5-(3-oxo-2,3,4,5-tetrahydro-6-pyridazinyl)indol

Analog Beispiel 2 a wurde 6-(4-Hydrazinophenyl)-2,3,4,5,-tetrahydro-pyridazin-3-on hergestellt, und die nach der Reduktion erhaltene Suspension ohne Isolierung des Hydrazins mit 4-Propionylpyridin versetzt. Nach 2 Stunden wurde der Rueckstand abgesaugt, nochmals in Wasser suspendiert, mit waessrigem Ammoniak neutralisiert und der Niederschlag abgesaugt. Man erhaelt als Rohprodukt 4-Propionyl-pyridin-[4-(3-oxo-2,3,4,5-tetrahydro-6-pyridazinyl)-phenylhydrazon] vom Schmp. 235-37 °C, welches ohne Reinigung analog Beispiel 1 in Polyphosphorsaeure zur Titelverbundung cyclisiert und durch Saeulenchromatographie (Laufmittel: Methylenchlorid/Methanol 98:2 bis 90:10) gereinigt wurde.
Ausbeute: 24 %, Schmp. > 300 °C aus Methanol.

Beispiel 9

2-(3-Pyridyl)-5-(3-oxo-2,3,4,5-tetrahydro-6-pyridazinyl)indol

Analog Beispiel 8 erhaelt man als Rohprodukt 3-Acetylpyridin-[4-(3-oxo-2,3,4,5-tetrahydro-6-pyridazinyl)-phenylhydrazon] vom Schmp. 236-37 °C (Ethanol), welches in Polyphosphorsaeure zur Titelverbindung cyclisiert wurde.
Ausbeute: 50 %, Schmp. 298-300 °C aus Ethanol

Beispiel 10

2-(4-Pyridazinyl)-5-(3-oxo-2,3,4,5-tetrahydro-6-pyridazinyl)indol

Analog Beispiel 8 erhaelt man als Rohprodukt 4-Acetyl-pyridazin-[4-(3-oxo-2,3,4,5-tetrahydro-6-pyridazinyl)phenylhydrazon] vom Schmp. 258-60 °C, welches ohne Reinigung in Polyphosphorsaeure zur Titelverbindung cyclisiert und ueber Saeulenchromatographie (Laufmittel: Methylenchlorid/Methanol 90:10) gereinigt wurde.
Ausbeute: 18 %, Schmp. > 300 °C aus Methanol.

Beispiel 11

2-(4-Thiazolyl)-5-(3-oxo-2,3,4,5-tetrahydro-6-pyridazinyl)indol

Analog Beispiel 8 erhaelt man als Rohprodukt 4-Acetyl-thiazol-[4-(3-oxo-2,3,4,5-tetrahydro-6-pyridazinyl)phenylhydrazon] vom Schmp. > 300 °C, welches ohne weitere Reinigung in Polyphosphorsaeure zur Titelverbindung cyclisiert wurde.
Ausbeute: 39 %, Schmp. 307-09 °C aus Methanol.

Beispiel 12

2-(2-Pyrazinyl)-5-(3-oxo-2,3,4,5,-tetrahydro-6-pyridazinyl)indol x 0.5 mol Ethanol

Analog Beispiel 8 erhaelt man als Rohprodukt 2-Acetyl-pyrazin-[4-(3-oxo-2,3,4,5-tetrahydro-6-pyridazinyl)phenylhydrazon] vom Schmp. 197-202°C, welches ohne weitere Reinigung in Polyphosphorsaeure zur Titelverbindung cyclisiert wurde.
Ausbeute: 8.4 %, Schmp. 284-86°C aus Ethanol.

Beispiel 13

2-Phenyl-5-(3-oxo-2,3,4,5-tetrahydro-6-pyridazinyl)indol

Analog Beispiel 8 erhaelt man als Rohprodukt Acetophenon-[4-(3-oxo-2,3,4,5-tetrahydro-6-pyridazinyl)-phenylhydrazon] vom Schmp. 176-78°C, welches ohne weitere Reinigung in Polyphosphorsaeure zur Titelverbindung cyclisiert und durch Saeulenchromatographie (Laufmittel: Methylenchlorid/Methanol 9:1) gereinigt wurde.
Ausbeute: 45 %, Schmp. 270-75°C.

Beispiel 14

2-(4-Methoxy-phenyl)-5-(3-oxo-2,3,4,5-tetrahydro-6-pyridazinyl)indol

Analog Beispiel 8 erhaelt man als Rohprodukt p-Methoxy-acetophenon-[4-(3-oxo-2,3,4,5-tetrahydro-6-pyridazinyl)phenylhydrazon] vom Schmp. 204-07°C, welches ohne weitere Reinigung in Polyphosphorsaeure zur Titelverbindung cyclisiert und durch Saeulenchromatographie (Laufmittel: Methylenchloride/Methanol 9:1) gereinigt wurde.
Ausbeute: 8 %, Schmp. 262-64°C.

Beispiel 15

2-(4-Methyl-phenyl)-5-(3-oxo-2,3,4,5-tetrahydro-6-pyridazinyl)indol

Analog Beispiel 8 erhaelt man als Rohprodukt 4-Methyl-acetophenon[4-(3-oxo-2,3,4,5-tetrahydro-6-pyridazinyl)phenylhydrazon] vom Schmp. 102-04°C, welches ohne weitere Reinigung in Polyphosphorsaeure zur Titelverbindung cyclisiert wurde.
Ausbeute: 5.1 %, Schmp. 266-68°C aus Ethanol.

Beispiel 16

2-(4-Methylmercapto-phenyl)-5-(3-oxo-2,3,4,5-tetrahydro-6-pyridazinyl)indol

Analog Beispiel 8 erhaelt man als Rohprodukt 4-Methylmercapto-acetophenon-[4-(3-oxo-2,3,4,5-tetrahydro-6-pyridazinyl)phenylhydrazon] vom Schmp. 126-28°C, welches ohne weitere Reinigung in Polyphosphorsaeure zur Titelverbindung cyclisiert wurde.
Ausbeute: 8.9 %, Schmp. 256-58°C aus Dioxan.

Beispiel 17

2-(2-Hydroxy-phenyl)-5-(3-oxo-2,3,4,5-tetrahydro-6-pyridazinyl)-indol x 0,5 mol Ethanol

Analog Beispiel 8 erhaelt man als Rohprodukt 2-Hydroxy-acetophenon-[4-(3-oxo-2,3,4,5-tetrahydro-6-pyridazinyl)phenylhydrazon] vom Schmp. 88-96°C, welches ohne weitere Reinigung in Polyphosphorsaeure zur Titelverbindung cyclisiert wurde. Das Rohprodukt wurde in Ethanol geloest, gekohlt und der eingedampfte Rueckstand mit Ether verrieben.
Ausbeute: 12.4 %, Schmp. 238-40°C.

Beispiel 18

2-[4-(Imidazol-1-yl)phenyl]-5-(3-oxo-2,3,4,5-tetrahydro-6-pyridazinyl)indol

Analog Beispiel 8 erhaelt man als Rohprodukt 4-(Imidazol-1-yl)-acetophenon-[4-(3-oxo-2,3,4,5-tetrahydro-6-pyridazinyl)phenylhydrazon] vom Schmp. > 300 ° C, welches ohne weitere Reinigung in Polyphosphorsaeure zur Titelverbindung cyclisiert und durch Saeulenchromatographie (Laufmittel: Methylenchlorid/ Methanol 95:5) gereinigt wurde.
Ausbeute: 25 %, Schmp. 295-98 ° C.

Beispiel 19

2-(4-Pyridyl)-5-(5-methyl-3-oxo-2,3,4,5-tetrahydro-6-pyridazinyl)indol

Ausgehend von 6-(4-Amino-phenyl)-5-methyl-2,3,4,5,-tetrahydropyridazin-3-on erhaelt man analog Beispiel 2 a 6-(4-Hydrazinophenyl)-5-methyl-2,3,4,5-tetrahydro-pyridazin-3-on, welches nach der Reduktion ohne Isolierung mit 4-Acetyl-pyridin versetzt wurde. Nach 3 h wurde der Rueckstand abgesaugt, nochmals in Wasser suspendiert, mit waessrigem Ammoniak neutralisiert und der Niederschlag abgesaugt und aus Ethanol umkristallisiert. Man erhaelt 65 % 4-Acetyl-pyridin-[4-(3-oxo-5-methyl-2,3,4,5-tetrahydro-6-pyridazinyl)phenylhydrazon] vom Schmp. 246-48 ° C (Ethanol). Das so erhaltene Hydrazone wurde analog Beispiel 1 in Polyphosphorsaeure zur Titelverbindung cyclisiert.
Ausbeute: 78 %, Schmp. 301-03 ° C aus Methanol.

Beispiel 20

2-(1,2,5-Thiadiazol-3-yl)-5-(5-methyl-3-oxo-2,3,4,5-tetrahydro-6-pyridazinyl)indol

Analog Beispiel 19 erhaelt man als Rohprodukt 3-Acetyl-1,2,5-thiadiazol-[4-(3-oxo-5-methyl-2,3,4,5-tetrahydro-6-pyridazinyl)phenylhydrazon] vom Schmp. 241-45 ° C, welches ohne weitere Reinigung in Polyphosphorsaeure zur Titelverbindung cyclisiert wurde.
Ausbeute: 48 %, Schmp. > 300 ° C aus Ethanol.

Beispiel 21

2-(4-Pyridyl)-3-methyl-5-(5-methyl-3-oxo-2,3,4,5-tetrahydro-6-pyridazinyl)indol

Analog Beispiel 19 erhaelt man 58 % 4-Propionyl-pyridin-[4-(3-oxo-5-methyl-2,3,4,5-tetrahydro-6-pyridazinyl)phenylhydrazon] vom Schmp. 233-35 ° C, welches in Polyphosphorsaeure zur Titelverbindung cyclisiert wurde.
Ausbeute: 53 %, Schmp. 294-97 ° C.

Beispiel 22

5-(3-Oxo-2,3,4,5-tetrahydro-6-pyridazinyl)indol-2-carbonsaeureethylester

3.02 g (10 mmol) Brenztraubensaeureethylester-[4-(oxo-2,3,4,5-tetrahydro-6-pyridazinyl)-phenylhydrazon] wurden analog Beispiel 1 in Polyphosphorsaeure zur Titelverbindung cyclisiert.
Ausbeute: 0.71 g (25 %), Schmp. 235-37 ° C (Diethylether).

Das Ausgangsmaterial laeßt sich wie folgt herstellen:

Zu einer Mischung aus 4.0 g (21 mmol) 6-(4-Aminophenyl)-2,3,4,5-tetrahydro-pyridazin-3-on, 5.1 ml konz. Salzsaeure und 25 g Eis tropft man unterhalb + 5 ° C Loesung aus 1.6 g Natriumnitrit und 5 ml Wasser. Man ruehrt 30 min bei 0 ° C nach und filtriert.

Zu einer Mischung aus 3.0 g (21 mmol) 2-Methylacetessigsaeureethylester und 25 g Eis tropft man gleichzeitig das vorstehende Filtrat sowie eine gekuehlte Loesung von 5.1 g Kaliumhydroxid in 13 ml Wasser. Man ruehrt 30 min bei 0 ° C nach, fuegt 20 g Eis und 3.9 ml konz. Salzsaeure zu und filtriert.

Der klebrige Rueckstand wird in Dichlormethan und wenig Methanol aufgenommen, ueber Natriumsulfat getrocknet, eingeengt und mit Ether verrieben. Es verbleiben 4.7 g (74 %) Brenztraubensaeureethylester-[4-(3-oxo-2,3,4,5-tetrahydro-6-pyridazinyl)phenylhydrazon vom Schmp.] 189-91 ° C.

Beispiel 23

5-(3-Oxo-2,3,4,5-tetrahydro-6-pyridazinyl)indol-2-carbonsaeure

3 g (10.5 mmol) des in Beispiel 22 erhaltenen Esters wurden mit 1.6 g Kaliumhydroxid und 25 ml 70 %igem Ethanol 2 h zum Rueckfluß erhitzt. Man engt im Vakuum ein, nimmt in Wasser auf, waescht mit Ether und stellt die waessrige Phase sauer. Es fallen 1.8 g (67 %) Titelverbindung vom Schmp. 185-87 °C aus.

Beispiel 24

2-(4-Pyridyl)-5-(2-oxo-1,2-dihydro-5-pyrazinyl)-indol x 1.5 mol $H_2O$

Ausgehend von 5-(4-Amino-phenyl)-1,2-dihydro-pyrazin-2-on erhaelt man analog Beispiel 2 a 5-(4-Hydrazinophenyl)-1,2-dihydro-pyrazin-2-on,welches nach der Reducktion ohne Isolierung mit 4-Acetylpyridin versetzt wurde. Nach 3 h wurde der Rueckstand abgesaugt, nochmals in Wasser suspendiert, mit waessrigem Ammoniak neutralisiert und der Niederschlag abgesaugt. Man erhaelt als Rohprodukt 4-Acetylpyridin-[4-(2-oxo-1,2-dihydro-5-pyrazinyl)phenylhydrazon] vom Schmp. 175-79 °C, welches ohne weitere Reinigung analog Beispiel 1 in Polyphosphorsaeure zur Titelverbindung cyclisiert wurde.
Ausbeute: 30 %, Schmp. > 300 °C aus Methanol.

Beispiel 25

2-(4-Pyridyl)-5-(2-oxo-2,3,-dihydro-6H-1,3,4-oxadiazin-5-yl)indol

a) Analog Beispiel 2 a erhaelt man ausgehend von 5-(4-Aminophenyl)-2,3-dihydro-6H-1,3,4-oxadiazin-2-on 64 % 5-(4-Hydrazinophenyl)-2,3-dihydro-6H-1,3,4-oxadiazin-2-on vom Schmp. 210-20 °C als Hydrochlorid.
b) 9.1 g (33 mmol) des nach a) erhaltenen Hydrazins wurden in 100 ml Methanol mit 3.5 ml (33 mmol) 4-Acetylpyridin versetzt und 1 h bei 25 °C geruehrt. Die ausgefallenen Kristalle wurden abgesaugt und mit wenig Methanol gewaschen. Man erhaelt 10 g (98 %) 4-Acetylpyridin-[4-(2-oxo-2,3-dihydro-6H-1,3,4-oxadiazin-5-yl)-phenylhydrazon] vom Schmp. 290-95 °C als Hydrochlorid.
c) Analog Beispiel 2 c wurden 10 g (30 mmol) des Hydrazons in Polyphosphorsaeure zur Titelverbindung cyclisiert.
Ausbeute: 6.9 g (73 %), Schmp. 322-25 °C aus Ethanol.

Beispiel 26

2-(4-Pyridyl)-5-(3-oxo-2,3,4,5-tetrahydro-1,2,4-triazin-6-yl)indol

a) Analog Beispiel 2 a erhaelt man ausgehend von 6-(4-Aminophenyl)-2,3,4,5-tetrahydro-1,2,4-triazin-3-on 65 % 6-(4-Hydrazinophenyl)-2,3,4,5-tetrahydro-1,2,4,-triazin-3-on vom Schmp. 249-59 °C als Hydrochlorid.
b) Analog Beispiel 25 b erhaelt man aus dem Hydrazin und 4-Acetylpyridin 63 % 4-Acetylpyridin-[4-(3-oxo-2,3,4,5-tetrahydro-1,2,4-triazin-6-yl)phenylhydrazon] vom Schmp. > 300 °C als Hydrochlorid.
c) Analog Beispiel 2 c wurden 5.5 g (18 mmol) des Hydrazons in Polyphosphorsaeure zur Titelverbindung cyclisiert und durch Saeulenchromatographie (Laufmittel: Methylenchlorid/Methanol/Eisessig 10:10:0.2) gereinigt. Die eingedampften Fraktionen wurden nochmals in Wasser suspendiert, mit waessrigem Ammoniak neutralisiert und abgesaugt.
Ausbeute: 1.8 g (35 %), Schmp. 313-16 °C.

Beispiel 27

2-(4-Pyridyl)-5-(5-oxo-4,5-dihydro-6H-1,3,4-oxadiazin-2-yl)indol x 1 mol Dimethylformamid

a) 3.2 g 1-(4-Nitrobenzoyl)-2-chloracetyl-hydrazin loeste man in 62.5 ml absoluten Dimethylformamid, gab 0.88 g einer 50 %igen Suspension von Natriumhydrid in Oel zu, ruehrte 15 min bei 120 °C, engte dann im Oelpumpenvakuum bei 60 °C zur Trockene ein, rieb den Rueckstand mit wenig Wasser durch, saugte die erhaltenen Kristalle ab und erhielt 2 g 2-(4-Nitrophenyl)-4,5-dihydro-6H-1,3,4-oxadiazin-5-on vom Schmp. 224-29 °C aus Methanol.

b) 1.5 g der nach a) erhaltenen Nitroverbindung wurden in 150 ml Methanol und 2.3 ml Eisessig mit 0.75 g 10 %igem Palladium auf Kohle bei 25 °C hydriert. Nach Abtrennen des Katalysators wurde das Filtrat eingedampft und mit Ether verrieben. Man erhaelt als Rohprodukt 1 g 2-(4-Aminophenyl)-4,5-dihydro-6H-1,3,4-oxadiazin-5-on vom Schmp. 238-45 °C.

c) Analog Beispiel 2 a wurde aus dem so erhaltenen Amin das 2-(4-Hydrazinophenyl)-4,5-dihydro-6H-1,3,4,-oxadiazin-5-on hergestellt und die nach der Reduktion erhaltene Suspension ohne Isolierung des Hydrazins mit 4-Acetylpyridin versetzt. Der Ansatz wurde 3 h bei 25 °C weitergeruehrt, der Niederschlag abgesaugt und mit Wasser gewaschen. Man erhaelt als Rohprodukt 4-Acetylpyridin-[4-(5-oxo-4,5-dihydro-6H-1,3,4-oxadiazin-2-yl)phenylhydrazon] vom Schmp. 195 °C.

d) Analog Beispiel 2 c wurden 0.7 g des nach c) erhaltenen Hydrazons in Polyphosphorsaeure zur Titelverbindung cyclisiert und in wenig Dimethylformamid geloest durch Saeulenchromatographie (Laufmittel: Essigester/Methanol 9:1) gereinigt.
Ausbeute: 90 mg, Schmp. 273-77 °C.

<u>Beispiel 28</u>

<u>2-(4-Pyridyl)-5-(4-methyl-5-oxo-4,5-dihydro-1,2,4-triazol-3-yl)indol</u>

a) 1.81 g 4-Nitrobenzoylhydrazid loeste man in 80 ml absolutem heißem Dioxan, versetzte bei 40 °C diese Loesung mit einer Loesung von 0.7 g Methylisocyanat in 5 ml Dioxan, ruehrte 30 min bei 25 °C nach, saugte das entstandene Kristallisat ab und erhielt so 2.2 g 1-(4-Nitrobenzoyl)-4-methyl-semicarbazid vom Schmp. 254-56 °C.

b) 2 g des Semicarbazids loeste man in 20 ml 1 N NaOH, erhitzte 7.5 Stunden auf 130 °C, neutralisierte nach dem Abkuehlen mit 2 N Salzsaeure, saugte das Kristallisat ab, wusch mit Wasser und Ethanol und erhielt so 1.3 g rohes 3-(4-Nitrophenyl)-4-methyl-1,2,4-triazol-5-on vom Schmp. 290-98 °C.

c) 2.2 g der nach b) erhaltenen Nitroverbindung hydrierte man, in 160 ml Methanol suspendiert, mit 0.6 g 10 %igem Palladium auf Kohle. Nach Abtrennen des Katalysators wurde das Filtrat im Vakuum eingedampft. Man erhielt so 1.6 g rohes 3-(4-Aminophenyl)-4-methyl-1,2,4-triazol-5-on vom Schmp. 228-30 °C.

d) Analog Beispiel 2 a wurde aus dem so erhaltenen Amin das 3-(4-Hydrazinophenyl)-4-methyl-1,2,4-triazol-5-on hergestellt und die nach der Reduktion erhaltene Suspension ohne Isolierung des Hydrazins mit 4-Acetylpyridin versetzt. Der Ansatz wurde 4 h bei 25 °C weitergeruehrt, der Niederschlag abgesaugt und mit Wasser und Ether nachgewaschen. Man erhielt so 2 g rohes 4-Acetylpyridin-[4-(5-oxo-4,5-dihydro-4-methyl-1,2,4-triazol-3-yl)phenylhydrazon] als Hydrochlorid.

e) Analog Beispiel 2 c wurde 1 g des nach d) erhaltenen Hydrazons in Polyphosphorsaeure zur Titelverbindung cyclisiert. Das Rohprodukt wurde wiederholt mit Ethanol ausgekocht, vom Ungeloesten noch heiß abgetrennt und in der Hitze mit Kohle behandelt. Das Filtrat wurde eingeengt und das dabei erhaltene Kristallisat abgesaugt und mit Ethanol und Ether gewaschen.
Ausbeute: 0.13 g, Schmp. > 300 °C.

<u>Beispiel 29</u>

<u>2-(4-Pyridyl)-5-(4,4-dimethyl-5-oxo-4,5-dihydro-3-pyrazolyl)indol</u>

a) Zu einer Suspension vom 9.6 g (0.2 mol) 50proz. Natriumhydrid in 30 ml abs. Dimethylformamid tropft man eine Loesung von 23.7 g (0.1 mol) 2-(4-Nitrobenzoyl)essigsaeureethylester in 100 ml Tetrahydrofuran, fuegt 12.5 ml Methyliodid zu und ruehrt 20 h bei Raumtemperatur. Danach gießt man in 1 l Wasser und extrahiert mit Ether, trocknet den Extrakt und engt ein. Es verbleiben 25.4 g 2-(4-Nitrobenzoyl) isobuttersaeureethylester als oeliges Rohprodukt.

b) Eine Mischung aus 17.0 g (64 mmol) des vorstehenden Rohprodukts, 9.0 ml Hydrazinhydrat und 150 ml Ethanol wird 18 h zum Rueckfluß erhitzt, anschließend im Vakuum eingeengt und an Kieselgel chromatographiert. Mit Dichlormethan:Methanol = 19:1 eluiert man 8.1 g (54 % d.Th.) 3,4-Dihydro-4,4-dimethyl-5-(4-nitrobenzoyl)-3-oxo-pyrazol als Oel.

c) 8.0 g (34 mmol) der vorstehenden Nitroverbindung werden in 200 ml Methanol ueber 1 g Raney-Nickel bei 1 bar Wasserstoffdruck hydriert. Nach 2 h wird filtriert, eingeengt und mit Ether verrieben. Man erhaelt 5.7 g 83 % d.Th.) 5-(4-Aminobenzoyl)-3,4-dihydro-4,4-dimethyl-3-oxo-pyrazol vom Schmp. 198-200 °C.

d) 3.0 g (15 mmol) der vorstehenden Aminoverbindung werden mit 60 ml 2 N Salzsaeure versetzt und

auf 0 °C gekuehlt. Hierzu tropft man eine Loesung von 1.2 g Natriumnitrit in 10 ml Wasser, ruehrt 5 min bei 0 °C nach, tropft eine Loesung aus 9.8 g Zinn(II)chlorid und 30 ml 2 N Salzsaeure zu, ruehrt 30 min bei Raumtemp. nach, filtriert und versetzt das Filtrat mit 2.5 ml 4-Acetyl-pyridin. Man ruehrt 5 h bei Raumtemperatur, laeßt ueber Nacht stehen, filtriert und trocknet den Niederschlag im Vakuum. Man erhaelt 5.7 g (98 % d.Th.) 4-Acetylpyridin-[4-(4,4-dimethyl-5-oxo-4,5-dihydro-3-pyrazolyl)-phenylhydrazon] als Dihydrochlorid vom Schmp. 271-274 °C.

e) 2.0 g (6mmol) des vorstehenden Hydrazons werden mit 50 g Polyphosphorsaeure 10 min bei 120 °C geruehrt. Man gießt auf Eis, stellt mit Ammoniak alkalisch, filtriert, nimmt den Niederschlag in Dichlormethan:Methanol = 9.1 auf, entfaerbt mit Tierkohle und engt ein. Nach Verreiben mit Ligroin erhaelt man die Titelverbindung.

Ausbeute: 0.34 g (16 %), Schmp. > 300 °C.

Beispiel 30

2-Methyl-3-phenyl-5-(3-oxo-2,3,4,5-tetrahydro-6-pyridazinyl)indol

4.8 g (20 mmol) 6-(4-Hydrazinophenyl)-2,3,4,5-tetrahydro-pyridazin-3-onHydrochlorid ( nach Beispiel 2 a) wurden in 75 ml 2 N Salzsaeure und 75 ml Ethanol suspendiert, mit 2.8 g (21 mmol) Phenylaceton versetzt, 3 h bei 25 °C geruehrt, abgesaugt und die Titelverbindung aus Methanol umkristallisiert.

Ausbeute: 4.85 g (80 %), Schmp. 297-99 °C.

Beispiel 31

2-(4-Pyridazinyl)-5-(5-methyl-3-oxo-2,3,4,5-tetrahydro-6-pyridazinyl)indol

Analog Beispiel 19 erhaelt man 70 % 4-Acetyl-pyridazin-[4-(3-oxo-5-methyl-2,3,4,5-tetrahydro-6-pyridazinyl)phenylhydrazon] vom Schmp. 265 °C (Zers.), welches in Polyphosphorsaeure zur Titelverbindung cyclisiert wurde.

Ausbeute: 29 % Schmp. 293-95 °C aus Methanol

**Patentansprüche**

**Patentansprüche für folgende Vertragsstaaten : BE, CH, DE, FR, GB, IT, LI, LU, NL, SE**

1. Indol-Derivate der Formel I

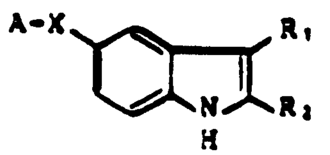

(I),

in welcher

$R_1$ Wasserstoff, die Methyl-, Ethyl-, Isopropyl-, n-Butyl-, Allyl-, Cyclohexyl-, Cyclopentenyl-, Cyan-, Ethoxycarbonyl- oder Phenylgruppe bedeutet,

$R_2$ den Pyrrolyl-, Furanyl-, Thiophenyl-, Pyrazolyl-, Imidazolyl-, Thiazolyl, Oxazolyl-, Triazolyl-, Tetrazolyl-, Thiadiazolyl-, Oxadiazolyl-, Pyridinyl-, N-Oxy-pyridinyl-, Pyrazinyl-, N,N-Dioxypyrazinyl-, Pyrimidinyl-, N,N-Dioxypyrimidinyl, Pyridazinyl-, Oxazinyl-, Thiazinyl-, Triazinyl- oder Tetrazinylrest darstellt, sowie deren durch Methyl, Ethyl, Methoxy, Ethoxy, Methylmercapto, Ethylmercapto und Chlorosubstituierten Derivate, oder den Phenylrest der allgemeinen Formel II

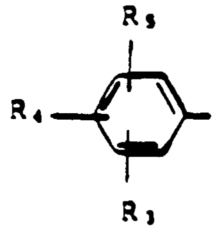

(II),

darstellt, wobei

$R_3$ ein Wasserstoff, die Methylsulfonyloxy-, Trifluormethylsulfonyloxy-, Methylsulfonylamino-, Trifluormethylsulfonylamino-, N-Methyl-methylsulfonylamino-, N-Methyl-trifluormethylsulfonylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethyloxy-, Methoxycarbonylmethyloxy-, Cyano-, Chloro-, Nitro-, Amino-, Dimethylamino-, Trifluormethyl- oder die 1-Imidazolylgruppe,

$R_4$ Wasserstoff, die Methyl-, Methoxy-, Dimethylamino- oder Chlorogruppe bedeutet,

$R_5$ Wasserstoff oder die Methoxygruppe ist,

A den 3-Oxo-2,3,4,5-tetrahydro-6-pyridazinyl-, 5-Methyl-3-oxo-2,3,4,5-tetrahydro-6-pyridazinyl-, 5-Hydroxymethyl-3-oxo-2,3,4,5-tetrahydro-6-pyridazinyl-, 3-Cyan-6-methyl-2-oxo-1,2-dihydro-5-pyridinyl-, 3-Aminocarbonyl-6-methyl-2-oxo-1,2-dihydro-5-pyridinyl-, 3-Oxo-2,3,4,5-tetrahydro-1,2,4-triazin-6-yl, 2-Oxo-4,5-dihydro-6H-1,3,4-oxadiazin-5-yl, 6-Oxo-1,4,5,6-tetrahydro-1,2,4-triazin-3-yl-, 5-Oxo-4,5-dihydro-6H-1,3,4-oxadiazin-2-yl-, 2-Oxo-1,2-di-hydro-5-pyrimidinyl-, 2-Oxo-1,2-dihydro-5-pyrazinyl-, 4,4-Dimethyl-5-oxo-4,5-dihydro-3-pyrazolyl-, 2-Oxo-4-pyrrolidinyl-, 4-Methyl-5-oxo-4,5-dihydro-1,2,4-triazol-3-yl- oder den 5(4)-Methyl-2-oxo-2,3-dihydro-4(5)-imidazolyl-rest und

X einen Valenzstrich, die Vinylengruppe oder die Ethylengruppe bedeutet,

deren Tautomere und deren physiologisch verträgliche Salze anorganischer und organischer Säuren

**2.** Indol-Derivate der Formel I gemäß Anspruch 1, in welcher

$R_1$ Wasserstoff, die Methyl-, Ethyl-, Isopropyl- oder Phenylgruppe bedeutet,

$R_2$ den Thiophenyl-, Thiazolyl-, Thiadiazolyl-, Pyridinyl-, Pyrazinyl-, Pyridazinylrest oder die Phenylgruppe, die durch eine Aminocarbonyl-, Chloro-, Trifluormethyl-, Methyl-, Methoxy-, Methylmercapto-, Hydroxy- oder 1-Imidazolylgruppe substituiert sein kann, bedeutet,

A den 3-Oxo-2,3,4,5-tetrahydro-6-pyridazinyl-, 5-Methyl-3-oxo-2,3,4,5-tetrahydro-6-pyridazinyl-, 2-Oxo-1,2-dihydro-5-pyrazinyl-, 2-Oxo-2,3-dihydro-6H-1,3,4-oxadiazin-5-yl-, 3-Oxo-2,3,4,5-tetrahydro-1,2,4-triazin-6-yl-, 5-Oxo-4,5-dihydro-6H-1,3,4-oxadiazin-2-yl-, 4-Methyl-5-oxo-4,5-dihydro-1,2,4-triazol-3-yl- oder 4,4-Dimethyl-5-oxo-4,5-dihydro-3-pyrazolylrest und

X einen Valenzstrich oder die Vinylengruppe bedeutet.

**3.** Verbindungen gemäß Anspruch 1 oder 2, mit der Bezeichnung
2-(4-Pyridyl)-5-(5-methyl-3-oxo-2,3,4,5-tetrahydro-6-pyridazinyl)-indol und
2-(4-Pyridyl)-5-(3-oxo-2,3,4,5-tetra-hydro-6-pyridazinyl)-indol.

**4.** Verfahren zur Herstellung von Verbindungen der Formel I, wie in den Ansprüchen 1-3 definiert, dadurch gekennzeichnet, daß man in an sich bekannter Weise eine Verbindung der Formel VI

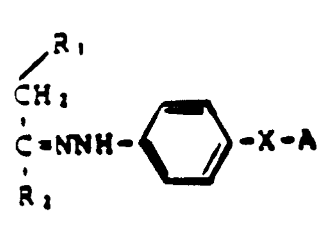

(VI),

in der $R_1$, $R_2$, A und X die oben angegebenen Bedeutungen haben, in einer Fischer-Indol-Synthese

cyclisiert und anschließend erhaltene Verbindungen der Formel I gewünschtenfalls in andere Verbindungen der Formel I umwandelt sowie ggf. die Verbindungen in pharmakologisch verträgliche Salze überführt.

**5.** Arzneimittel enthaltend eine Verbindung gemäß Anspruch 1, 2 oder 3 neben üblichen pharmakologischen Träger- und/oder Hilfsstoffen.

**6.** Verbindungen gemäß Anspruch 1, 2 oder 3 zur Prophylaxe bzw. Behandlung von Herz- und Kreislauferkrankungen.

**7.** Verwendung von Verbindungen gemäß Anspruch 1, 2 oder 3 zur Herstellung von Arzneimitteln zur Prophylaxe oder Behandlung von Herz- und Kreislauferkrankungen.

**8.** Verbindungen der Formel VI

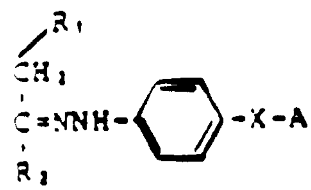

(VI),

in welcher $R_1$, $R_2$, X und A die in Anspruch 1 angegebene Bedeutungen haben.

**Patentansprüche für folgenden Vertragsstaat : AT**

**1.** Verfahren zur Herstellung von Indol-Derivate der Formel I

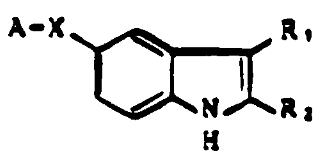

(I),

in welcher

$R_1$ Wasserstoff, die Methyl-, Ethyl-, Isopropyl-, n-Butyl-, Allyl-, Cyclohexyl-, Cyclopentenyl-, Cyan-, Ethoxycarbonyl- oder Phenylgruppe bedeutet,

$R_2$ den Pyrrolyl-, Furanyl-, Thiophenyl-, Pyrazolyl-, Imidazolyl-, Thiazolyl-, Oxazolyl-, Triazolyl-, Tetrazolyl-, Thiadiazolyl-, Oxadiazolyl-, Pyridinyl-, N-Oxy-pyridinyl-, Pyrazinyl-, N,N-Dioxypyrazinyl-, Pyrimidinyl-, N,N-Dioxypyrimidinyl, Pyridazinyl-, Oxazinyl-, Thiazinyl-, Triazinyl- oder Tetrazinylrest darstellt, sowie deren durch Methyl, Ethyl, Methoxy, Ethoxy, Methylmercapto, Ethylmercapto und Chlorosubstituierten Derivate,
oder den Phenylrest der allgemeinen Formel II

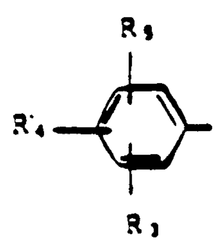

(II),

darstellt, wobei

$R_3$ ein Wasserstoff, die Methylsulfonyloxy-, Trifluormethylsulfonyloxy-, Methylsulfonylamino-, Trifluormethylsulfonylamino-, N-Methyl-methylsulfonylamino-, N-Methyl-trifluormethylsulfonylamino-, Methylsulfenylmethyl-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfonyl-, Hydroxy-, Methyl-, Methoxy-, Propargyloxy-, Cyanmethyloxy-, Methoxycarbonylmethyloxy-, Cyano-, Chloro-, Nitro-, Amino-, Dimethylamino-, Trifluormethyl- oder die 1-Imidazolylgruppe,

$R_4$ Wasserstoff, die Methyl-, Methoxy-, Dimethylamino- oder Chlorogruppe bedeutet,

$R_5$ Wasserstoff oder die Methoxygruppe ist,

A den 3-Oxo-2,3,4,5-tetrahydro-6-pyridazinyl-, 5-Methyl-3-oxo-2,3,4,5-tetrahydro-6-pyridazinyl-, 5-Hydroxymethyl-3-oxo-2,3,4,5-tetrahydro-6-pyridazinyl-, 3-Cyan-6-methyl-2-oxo-1,2-dihydro-5-pyridinyl-, 3-Aminocarbonyl-6-methyl-2-oxo-1,2-dihydro-5-pyridinyl-, 3-Oxo-2,3,4,5-tetrahydro-1,2,4-triazin-6-yl, 2-Oxo-4,5-dihydro-6H-1,3,4-oxadiazin-5-yl, 6-Oxo-1,4,5,6-tetrahydro-1,2,4-triazin-3-yl-, 5-Oxo-4,5-dihydro-6H-1,3,4-oxadiazin-2-yl-, 2-Oxo-1,2-di-hydro-5-pyrimidinyl-, 2-Oxo-1,2-dihydro-5-pyrazinyl-, 4,4-Dimethyl-5-oxo-4,5-dihydro-3-pyrazolyl-, 2-Oxo-4-pyrrolidinyl-, 4-Methyl-5-oxo-4,5-dihydro-1,2,4-triazol-3-yl- oder den 5(4)-Methyl-2-oxo-2,3-dihydro-4(5)-imidazolyl-rest und

X einen Valenzstrich, die Vinylengruppe oder die Ethylengruppe bedeutet,

dadurch gekennzeichnet, daß man in an sich bekannter Weise eine Verbindung der Formel VI

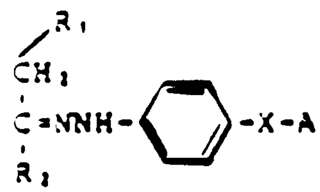

(VI),

in der $R_1$, $R_2$, A und X die oben angegebenen Bedeutungen haben, in einer Fischer-Indol-Synthese cyclisiert und anschließend erhaltene Verbindungen der Formel I gewünschtenfalls in andere Verbindungen der Formel I umwandelt sowie ggf. die Verbindungen in pharmakologisch verträgliche Salze überführt.

2. Verfahren gemäß Anspruch 1 zur Herstellung von Verbindungen der Formel I, in welcher

$R_1$ Wasserstoff, die Methyl-, Ethyl-, Isopropyl- oder Phenylgruppe bedeutet,

$R_2$ den Thiophenyl-, Thiazolyl-, Thiadiazolyl-, Pyridinyl-, Pyrazinyl-, Pyridazinylrest oder die Phenylgruppe, die durch eine Aminocarbonyl-, Chloro-, Trifluormethyl-, Methyl-, Methoxy-, Methylmercapto-, Hydroxy- oder 1-Imidazolylgruppe substituiert sein kann, bedeutet,

A den 3-Oxo-2,3,4,5-tetrahydro-6-pyridazinyl-, 5-Methyl-3-oxo-2,3,4,5-tetrahydro-6-pyridazinyl-, 2-Oxo-1,2-dihydro-5-pyrazinyl-, 2-Oxo-2,3-dihydro-6H-1,3,4-oxadiazin-5-yl-, 3-Oxo-2,3,4,5-tetrahydro-1,2,4-triazin-6-yl-, 5-Oxo-4,5-dihydro-6H-1,3,4-oxadiazin-2-yl, 4-Methyl-5-oxo-4,5-dihydro-1,2,4-triazol-3-yl- oder 4,4-Dimethyl-5-oxo-4,5-dihydro-3-pyrazolylrest und

X einen Valenzstrich oder die Vinylengruppe bedeutet.

3. Verfahren gemäß Anspruch 1 oder 2 zur Herstellung von 2-(4-Pyridyl)-5-(5-methyl-3-oxo-2,3,4,5-tetrahydro-6-pyridazinyl)-indol und 2-(4-Pyridyl)-5-(3-oxo-2,3,4,5-tetrahydro-6-pyridazinyl)-indol.

4. Verfahren zur Herstellung der Verbindungen der Formel VI

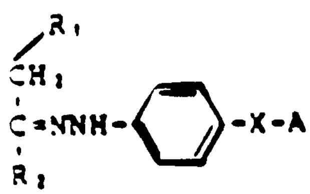

(VI),

in der $R_1$, $R_2$, X und A die in Anspruch 1 angegebene Bedeutung haben,
dadurch gekennzeichnet, daß man nach an sich bekannten Methoden Amine der Formel III

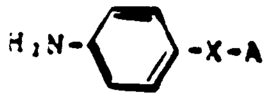

(III),

in der X und A die in Anspruch 10 angegebenen Bedeutungen haben,

A) diazotiert, anschließend entweder

a) zu den Hydrazinen der Formel IV in der X und A die angegebenen Bedeutungen haben, reduziert und mit Verbindungen der Formel V

$R_2COCH_2R_1$ (V),

in der $R_1$ und $R_2$ die angegebenen Bedeutungen haben, umsetzt oder

b) in einer Japp-Klingemann-Reaktion mit einer Verbindung der Formel VII

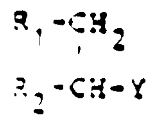

(VII),

in der $R_1$ und $R_2$ die angegebenen Bedeutungen haben und Y einen aktivierenden Rest darstellt, umsetzt und anschließend verseift, oder

B) mit Halogenessigestern VIII

$Z-CH_2-COOEt$ (VIII),

in der Z Halogen bedeutet, umsetzt, anschließend verseift und zu den Verbindungen IX

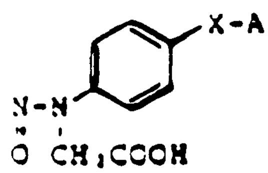

(IX)

in denen X und A die angegebenen Bedeutungen haben nitrosiert, diese mit wasserentziehenden Reagenzien zu den Sydnonen X

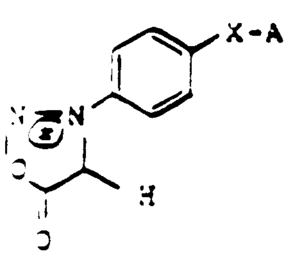

(X)

in denen X und A die angegebenen Bedeutungen haben umsetzt, die mit den Ketonen V

$R_2COCH_2R_1$ (V),

in denen $R_1$ und $R_2$ die oben angegebene Bedeutung haben, zu Verbindungen der Formel VI

umgesetzt werden.

5. Verfahren zur Herstellung von Arzneimitteln enthaltend Verbindungen der Formel I wie in Anspruch 1 definiert, dadurch gekennzeichnet, daß man diese Verbindungen zusammen mit pharmakologischen Träger- und/oder Hilfstoffen zu Arzneimitteln verarbeitet.

**Claims**

**Claims for the following Contracting States : BE, CH, DE, FR, GB, IT, LI, LU, NL, SE**

1. Indole derivatives of the formula I

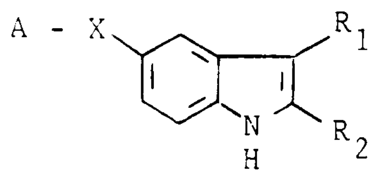

(I),

in which $R_1$ signifies hydrogen, the methyl, ethyl, isopropyl, n-butyl, allyl, cyclohexyl, cyclopentenyl, cyano, ethoxycarbonyl or phenyl group, $R_2$ represents the pyrrolyl, furanyl, thiophenyl, pyrazolyl, imidazolyl, thiazolyl, oxazolyl, triazolyl, tetrazolyl, thiadiazolyl, oxadiazolyl, pyridinyl, N-oxypyridinyl, pyrazinyl, N,N-dioxypyrazinyl, pyrimidinyl, N,N-dioxypyrimidinyl, pyridazinyl, oxazinyl, thiazinyl, triazinyl or tetrazinyl radical, as well as their derivatives substituted by methyl, ethyl, methoxy, ethoxy, methylmercapto, ethylmercapto and chloro, or the phenyl radical of the general formula II

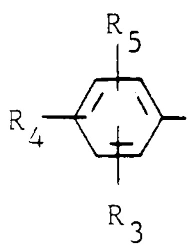

(II),

whereby $R_3$ signifies a hydrogen, the methylsulphonyloxy, trifluoromethylsulphonyloxy, methylsulphonylamino, trifluoromethylsulphonylamino, N-methyl-methylsulphonylamino, N-methyl-trifluoromethylsulphonylamino, methylsulphenylmethyl, methylsulphinylmethyl, methylsulphonylmethyl, aminocarbonyl, aminosulphonyl, methylaminosulphonyl, dimethylaminosulphonyl, acetylamino, methylmercapto, methylsulphonyl, hydroxyl, methyl, methoxy, propargyloxy, cyanomethyloxy, methoxycarbonylmethyloxy, cyano, chloro, nitro, amino, dimethylamino, trifluoromethyl or the 1-imidazolyl group, $R_4$ hydrogen, the methyl, methoxy, dimethylamino or chloro group, $R_5$ is hydrogen or the methoxy group, A signifies the 3-oxo-2,3,4,5-tetrahydro-6-pyridazinyl, 5-methyl-3-oxo-2,3,4,5-tetrahydro-6-pyridazinyl, 5-hydroxymethyl-3-oxo-2,3,4,5-tetrahydro-6-pyridazinyl, 3-cyano-6-methyl-2-oxo-1,2-dihydro-5-pyridinyl, 3-aminocarbonyl-6-methyl-2-oxo-1,2-dihydro-5-pyridinyl, 3-oxo-2,3,4,5-tetrahydro-1,2,4-triazin-6-yl, 2-oxo-4,5-dihydro-6H-1,3,4-oxadiazin-5-yl, 6-oxo-1,4,5,6-tetrahydro-1,2,4-triasin-3-yl, 5-oxo-4,5-dihydro-6H-1,3,4-oxadiazin-2-yl, 2-oxo-1,2-dihydro-5-pyrimidinyl, 2-oxo-1,2-dihydro-5-pyrazinyl, 4,4-dimethyl-5-oxo-4,5-dihydro-3-pyrasolyl, 2-oxo-4-pyrrolidinyl, 4-methyl-5-oxo-4,5-dihydro-1,2,4-triazol-3-yl or the 5-(4)-methyl-2-oxo-2,3-dihydro-4(5)-imidazolyl radical and X a valency bond, the vinylene group or the ethylene group, their tautomers and their physiologically compatible salts of inorganic and organic acids.

2. Indole derivatives of the formula I according to claim 1, in which $R_1$ signifies hydrogen, the methyl, ethyl, isopropyl or phenyl group, $R_2$ signifies the thiophenyl, thiazolyl, thiadiazolyl, pyridinyl, pyrazinyl, pyridazinyl radicals or the phenyl group which can be substituted by an aminocarbonyl, chloro, trifluoromethyl, methyl, methoxy, methylmercapto, hydroxyl or 1-imidazolyl group, A signifies the 3-oxo-2,3,4,5-tetrahydro-6-pyridazinyl, 5-methyl-3-oxo-2,3,4,5-tetrahydro-6-pyridazinyl, 2-oxo-1,2-dihydro-5-pyrazinyl, 2-oxo-2,3-dihydro-6H-1,3,4-oxadiazin-5-yl, 3-oxo-2,3,4,5-tetrahydro-1,2,4-triazin-6-yl, 5-oxo-4,5-dihydro-6H-1,3,4-oxadiazin-2-yl, 4-methyl-5-oxo-4,5-dihydro-1,2,4-triazol-3-yl or 4,4-dimethyl-5-oxo-4,5-dihydro-3-pyrazolyl radical and X a valency bond or the vinylene group.

**3.** Compounds according to claim 1 or 2 with the designation 2-(4-pyridyl)-5-(5-methyl-3-oxo-2,3,4,5-tetrahydro-6-pyridazinyl)-indole and 2-(4-pyridyl)-5-(3-oxo-2,3,4,5-tetrahydro-6-pyridazinyl)-indole.

**4.** Process for the preparation of compounds of the formula I as defined in claims 1 - 3, characterised in that, in per se known way, one cyclises a compound of the formula VI

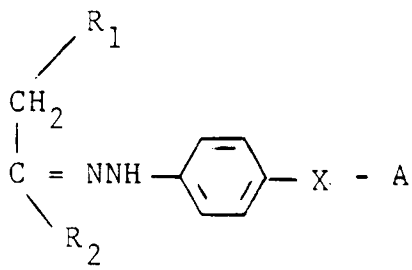

(VI)

in which $R_1$, $R_2$, A and X have the above-given meanings, in a Fischer indole synthesis and subsequently, if desired, converts compounds obtained of the formula I into other compounds of the formula I, as well as possibly converts the compounds into pharmacologically compatible salts.

**5.** Medicaments containing a compound according to claim 1, 2 or 3, besides usual pharmacological carrier and/or adjuvant materials.

**6.** Compounds according to claim 1, 2 or 3 for the prophylaxis or treatment of heart and circulatory diseases.

**7.** Use of compounds according to claim 1, 2 or 3 for the preparation of medicaments for the prophylaxis or treatment of heart and circulatory diseases.

**8.** Compounds of the formula VI

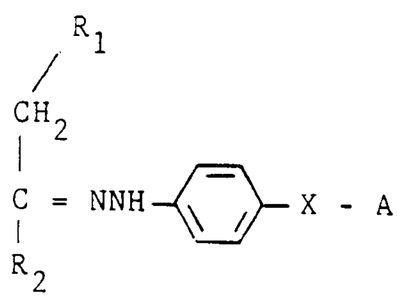

(VI)

in which $R_1$, $R_2$, X and A have the meanings given in claim 1.

**Claims for the following Contracting State : AT**

**1.** Process for the preparation of indole derivatives of the formula I

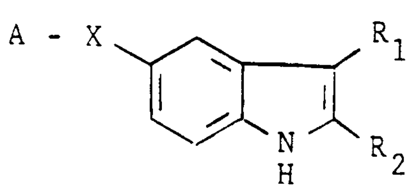

(I),

in which $R_1$ signifies hydrogen, the methyl, ethyl, isopropyl, n-butyl, allyl, cyclohexyl, cyclopentenyl, cyano, ethoxycarbonyl or phenyl group, $R_2$ represents the pyrrolyl, furanyl, thiophenyl, pyrazolyl, imidazolyl, thiazolyl, oxazolyl, triazolyl, tetrazolyl, thiadiazolyl, oxadiazolyl, pyridinyl, N-oxypyridinyl, pyrazinyl, N,N-dioxypyrazinyl, pyrimidinyl, N,N-dioxypyrimidinyl, pyridazinyl, oxazinyl, thiazinyl, triazinyl or tetrazinyl radical, as well as their derivatives substituted by methyl, ethyl, methoxy, ethoxy, methylmercapto, ethylmercapto and chloro, or the phenyl radical of the general formula II

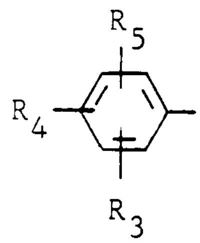

(II)

whereby $R_3$ signifies a hydrogen, the methylsulphonyloxy, trifluoromethylsulphonyloxy, methylsulphonylamino, trifluoromethylsulphonylamino, N-methyl-methylsulphonylamino, N-methyl-trifluoromethylsulphonylamino, methylsulphenylmethyl, methylsulphinylmethyl, methylsulphonylmethyl, aminocarbonyl, aminosulphonyl, methylaminosulphonyl, dimethylaminosulphonyl, acetylamino, methylmercapto, methylsulphonyl, hydroxyl, methyl, methoxy, propargyloxy, cyanomethyloxy, methoxycarbonylmethyloxy, cyano, chloro, nitro, amino, dimethylamino, trifluoromethyl or the 1-imidazolyl group, $R_4$ hydrogen, the methyl, methoxy, dimethylamino or chloro group, $R_5$ is hydrogen or the methoxy group, A signifies the 3-oxo-2,3,4,5-tetrahydro-6-pyridazinyl, 5-methyl-3-oxo-2,3,4,5-tetrahydro-6-pyridazinyl, 5-hydroxymethyl-3-oxo-2,3,4,5-tetrahydro-6-pyridazinyl, 3-cyano-6-methyl-2-oxo-1,2-dihydro-5-pyridinyl, 3-aminocarbonyl-6-methyl-2-oxo-1,2-dihydro5-pyridinyl, 3-oxo-2,3,4,5-tetrahydro-1,2,4-triazin-6-yl, 2-oxo-4,5-dihydro-6H-1,3,4-oxadiazin-5-yl, 6-oxo-1,4,5,6-tetrahydro-1,2,4-triazin-3-yl, 5-oxo-4,5-dihydro-6H -1,3,4-oxadiazin-2-yl, 2-oxo-1,2-dihydro-5-pyrimidinyl, 2-oxo-1,2-dihydro-5-pyrazinyl, 4,4-dimethyl-5-oxo-4,5-dihydro-3-pyrazolyl, 2-oxo-4-pyrrolidinyl,4-methyl-5-oxo-4,5-dihydro-1,2,4-triazol-3-yl or the 5(4)-methyl-2-oxo-2,3-dihydro-4(5)-imidazolyl radical and X a valency bond, the vinylene group or the ethylene group, characterised in that, in per se known way, one cyclises a compound of the formula VI

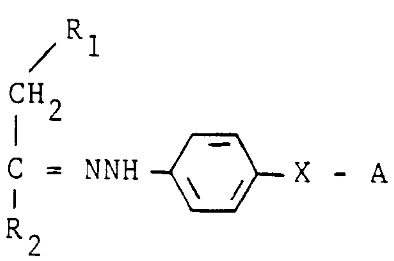

(VI),

in which $R_1$, $R_2$, A and X have the above-given meanings, in a Fischer indole synthesis and subsequently, if desired, converts the compounds obtained of the formula I into other compounds of the formula I, as well as possibly converts the compounds into pharmacologically compatible salts.

**2.** Process according to claim 1 for the preparation of compounds of the formula I, in which $R_1$ signifies hydrogen, the methyl, ethyl, isopropyl or phenyl group, $R_2$ signifies the thiophenyl, thiazolyl, thiadiazolyl, pyridinyl, pyrazinyl, pyridazinyl radical or the phenyl group which can be substituted by an aminocarbonyl, chloro, trifluoromethyl, methyl, methoxy, methylmercapto, hydroxyl or 1-imidazolyl group, A signifies the 3-oxo-2,3,4,5-tetrahydro-6-pyridazinyl, 5-methyl-3-oxo-2,3,4,5-tetrahydro-6-pyridazinyl, 2-oxo-1,2-dihydro-5-pyrazinyl, 2-oxo-2,3-dihydro-6H-1,3,4-oxadiazin-5-yl, 3-oxo-2,3,4,5-tetrahydro-1,2,4-triazin-6-yl, 5-oxo-4,5-dihydro-6H-1,3,4-oxadiazin-2-yl, 4-methyl-5-oxo-4,5-dihydro-1,2,4-triazol-3-yl or 4,4-dimethyl-5-oxo-4,5-dihydro-3-pyrazolyl radical and X a valency bond or the vinylene group.

**3.** Process according to claim 1 or 2 for the preparation of 2-(4-pyridyl)-5-(5-methyl-3-oxo-2,3,4,5-tetrahydro-6-pyridazinyl)-indole and 2-(4-pyridyl)-5-(3-oxo-2,3,4,5-tetrahydro-6-pyridazinyl)-indole.

**4.** Process for the preparation of compounds of the formula VI

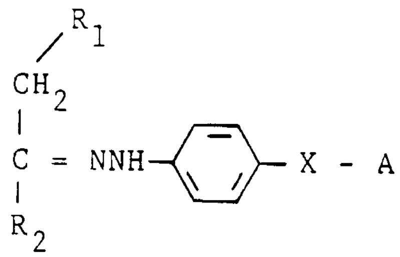

(VI),

in which $R_1$, $R_2$, X and A have the meaning given in claim 1, characterised in that, according to per se known methods, amines of the formula III

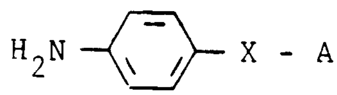

(III),

in which X and A have the meanings given in claim 1, are

A) diazotised, subsequently either

a) reduced to the hydrazines of the formula IV, in which X and A have the given meanings, and reacted with compounds of the formula V

$R_2COCH_2R_1$ (V)

in which $R_1$ and $R_2$ have the given meanings, or

b) reacted in the Japp-Klingemann reaction with a compound of the formula VII

$R_1 - CH_2$

$R_2 - CH - Y$ (VII),

in which $R_1$ and $R_2$ have the given meanings and Y represents an activating residue, or

B) reacted with haloacetic esters VIII

$Z-CH_2-COOEt$ (VIII),

in which Z signifies halogen, subsequently saponified and nitrosated to the compounds IX

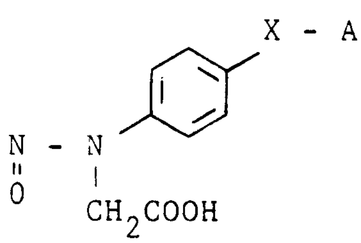

(IX)

in which X and A have the given meanings, these reacted with water-removing reagents to the sydnones X

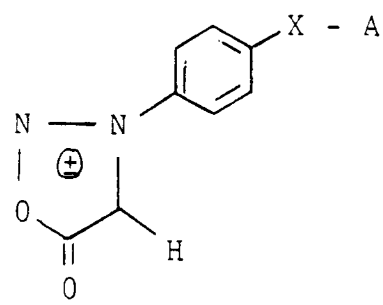

(X)

in which X and A have the given meanings, which are reacted with the ketones V

$R_2COCH_2R_1$ (V)

in which $R_1$ and $R_2$ have the above-given meanings, to compounds of the formula VI.

**5.** Process for the preparation of medicaments containing compounds of the formula I as defined in claim 1, characterised in that one works up these compounds, together with pharmacological carrier and/or adjuvant materials, to medicaments.

**Revendications**

**Revendications pour les Etats contractants suivants : BE, CH, DE, FR, GB, IT, LI, LU, NL, SE**

**1.** Dérivés d'indole de formule I

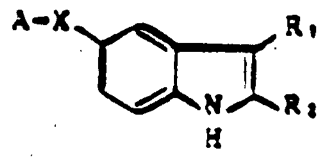

(I),

dans laquelle

$R_1$ représente l'hydrogène, le groupe méthyle, éthyle, isopropyle, n-butyle, allyle, cyclohexyle, cyclopentényle, cyano, éthoxycarbonyle ou phényle,

$R_2$ représente le groupe pyrrolyle, furanyle, thiophényle, pyrazolyle, imidazolyle, thiazolyle, oxazolyle, triazolyle, tétrazolyle, thiadiazolyle, oxadiazolyle, pyridinyle, N-oxypyridinyle, pyrazinyle, N,N-dioxypyrazinyle, pyrimidinyle, N,N-dioxypyrimidinyle, pyridazinyle, oxazinyle, thiazinyle, triazinyle ou tétrazinyle, ainsi que leurs dérivés substitués par un groupe méthyle, éthyle, méthoxy, éthoxy, méthylmercapto, éthylmercapto et chloro, ou le groupe phényle de formule générale II

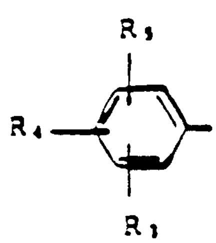

(II),

dans laquelle

$R_3$ représente l'hydrogène, le groupe méthylsulfonyloxy, trifluorométhylsulfonyloxy, méthylsulfonylamino, trifluorométhylsulfonylamino, N-méthyl-méthylsulfonylamino, N-méthyltrifluorométhylsulfonylamino, méthylsulfénylméthyle, méthylsulfinylméthyle, méthylsulfonylméthyle, aminocarbonyle, aminosulfonyle, méthylaminosulfonyle, diméthylaminosulfonyle, acétylamino,

méthylmercapto, méthylsulfonyle, hydroxy, méthyle, méthoxy, propargyloxy, cyanométhyloxy, méthoxycarbonylméthyloxy, cyano, chloro, nitro, amino, diméthylamino, trifluorométhyle et 1-imidazolyle,

$R_4$ représente l'hydrogène, le groupe méthyle, méthoxy, diméthylamino ou

$R_5$ représente l'hydrogène ou le groupe méthoxy,

A représente le groupe 3-oxo-2,3,4,5-tétrahydro-6-pyridazinyle, 5-méthyl-3-oxo-2,3,4,5-tétrahydro-6-pyridazinyle, 5-hydroxyméthyl-3-oxo-2,3,4,5-tétrahydro-6-pyridazinyle, 3-cyano-6-méthyl-2-oxo-1,2-dihydro-5-pyridinyle, 3-aminocarbonyl-6-méthyl-2-oxo-1,2-dihydro-5-pyridinyle, 3-oxo-2,3,4,5-tétrahydro-1,2,4-triazine-6-yle, 2-oxo-4,5-dihydro-6H-1,3,4-oxadiazine-5-yle, 6-oxo-1,4,5,6-tétrahydro-1,2,4-triazine-3-yle, 5-oxo-4,5-dihydro-6H-1,3,4 oxadiazine-2-yle, 2-oxo-1,2-di-hydro-5-pyrimidinyle, 2-oxo-1,2-dihydro-5-pyrazinyle, 4,4-diméthyl-5-oxo-4,5-dihydro-3-pyrazolyle, 2-oxo-4-pyrrolidinyle, 4-méthyl-5-oxo-4,5-dihydro-1,2,4-triazol-3-yle, ou 5(4)-méthyl-2-oxo-2,3-dihydro-4(5)-imidazolyle et

X représente une valence libre, le groupe vinylène ou le groupe éthylène,

leurs tautomères et leurs sels formés avec des acides minéraux et organiques, physiologiquement acceptables.

**2.** Dérivés d'indole de formule I selon la revendication 1, dans laquelle
$R_1$ représente l'hydrogène, le groupe méthyle, éthyle, isopropyle ou phényle,
$R_2$ représente le groupe thiophényle, thiazolyle, thiadiazolyle, pyridinyle, pyrazinyle, pyridazinyle ou le groupe phényle, qui peut être substitué par un groupe aminocarbonyle, chloro, trifluorométhyle, méthyle, méthoxy, méthylmercapto, hydroxy ou 1-imidazolyle,
A représente le groupe 3-oxo-2,3,4,5-tétrahydro-6-pyridazinyle, 5-méthyl-3-oxo-2,3,4,5-tétrahydro-6-pyridazinyle, 2-oxo-1,2-dihydro-5-pyrazinyle, 2-oxo-2,3-dihydro-6H-1,3,4-oxadiazine-5-yle, 3-oxo-2,3,4,5-tétrahydro-1,2,4-triazine-6-yle, 5-oxo-4,5-dihydro-6H-1,3,4-oxadiazine-2-yle, 4-méthyl-5-oxo-4,5-dihydro-1,2,4-triazol-3-yle ou 4,4-diméthyl-5-oxo-4,5-dihydro-3-pyrazolyle et
X représente une valence libre ou le groupe vinylène.

**3.** Composés selon la revendication 1 ou 2, qui sont les
2-(4-pyridyl)-5-(5-méthyl-3-oxo-2,3,4,5-tétrahydro-6-pyridazinyl)-indole et
2-(4-pyridyl)-5-(3-oxo-2,3,4,5-tétrahydro-6-pyridazinyl)-indole.

**4.** Procédé de préparation des composés de formule I, tels que définis dans les revendications 1 à 3, caractérisé en ce que, de manière connue en soi, on cyclise un composé de formule VI

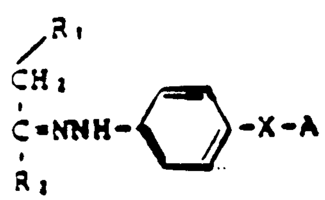

(VI) ,

dans laquelle $R_1$, $R_2$, A et X ont les significations mentionnées ci-dessus, selon une synthèse d'indole de Fischer, et ensuite, on transforme éventuellement les composés de formule I obtenus en d'autres composés de formule I, ainsi qu'éventuellement, on transforme les composés en leurs sels pharmacologiquement acceptables.

**5.** Médicament contenant un composé selon la revendication 1, 2 ou 3, en plus de véhicules et/ou d'adjuvants pharmacologiques usuels.

**6.** Composés selon la revendication 1, 2 ou 3, pour la prophylaxie ou le traitement des affections cardiovasculaires.

**7.** Utilisation des composés selon la revendication 1, 2 ou 3 pour la préparation de médicaments destinés à la prophylaxie ou au traitement des affections cardiovasculaires.

**8.** Composés de formule VI

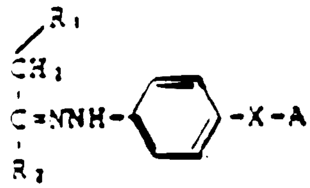 (VI),

dans laquelle $R_1$, $R_2$, X et A ont la signification mentionnée dans la revendication 1.

**Revendications pour l'Etat contractants suivants : AT**

**1.** Procédé pour la préparation de dérivés d'indole de formule I

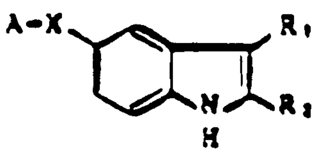 (I),

dans laquelle

$R_1$ représente l'hydrogène, le groupe méthyle, éthyle, isopropyle, n-butyle, allyle, cyclohexyle, cyclopentényle, cyano, éthoxycarbonyle ou phényle,

$R_2$ représente le groupe pyrrolyle, furanyle, thiophényle, pyrazolyle, imidazolyle, thiazolyle, oxazolyle, triazolyle, tétrazolyle, thiadiazolyle, oxadiazolyle, pyridinyle, N-oxypyridinyle, pyrazinyle, N,N-dioxypyrazinyle, pyrimidinyle, N,N-dioxypyrimidinyle, pyridazinyle, oxazinyle, thiazinyle, triazinyle ou tétrazinyle, ainsi que leurs dérivés substitués par un groupe méthyle, éthyle, méthoxy, éthoxy, méthylmercapto, éthylmercapto et chloro, ou le groupe phényle de formule générale II

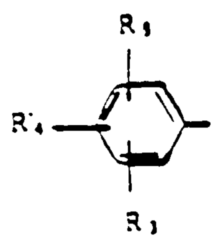 (II),

dans laquelle

$R_3$ représente l'hydrogène, le groupe méthylsulfonyloxy, trifluorométhylsulfonyloxy, méthylsulfonylamino, trifluorométhylsulfonylamino, N-méthyl-méthylsulfonylamino, N-méthyltrifluorométhylsulfonylamino, méthylsulfénylméthyle, méthylsulfinylméthyle, méthylsulfonylméthyle, aminocarbonyle, aminosulfonyle, méthylaminosulfonyle, diméthylaminosulfonyle, acétylamino, méthylmercapto, méthylsulfonyle, hydroxy, méthyle, méthoxy, propargyloxy, cyanométhyloxy, méthoxycarbonylméthyloxy, cyano chloro, nitro, amino, diméthylamino, trifluorométhyle et 1-imidazolyle,

$R_4$ représente l'hydrogène, le groupe méthyle, méthoxy, diméthylamino ou

$R_5$ représente l'hydrogène ou le groupe méthoxy,

A représente le groupe 3-oxo-2,3,4,5-tétrahydro-6-pyridazinyle, 5-méthyl-3-oxo-2,3,4,5-tétrahydro-6-pyridazinyle, 5-hydroxyméthyl-3-oxo-2,3,4,5-tétrahydro-6-pyridazinyle, 3-cyano-6-méthyl-2-oxo-1,2-dihydro-5-pyridinyle, 3-aminocarbonyl-6-méthyl-2-oxo-1,2-dihydro-5-pyridinyle, 3-oxo-2,3,4,5-tétrahydro-1,2,4-triazine-6-yle, 2-oxo-4,5-dihydro-6H-1,3,4-oxadiazine-5-yle, 6-oxo-1,4,5,6-tétrahydro-1,2,4-triazine-3-yle, 5-oxo-4,5-dihydro-6H-1,3,4-oxadiazine-2-yle, 2-oxo-1,2-di-hydro-5-pyrimidinyle, 2-oxo-1,2-dihydro-5-pyrazinyle, 4,4-diméthyl-5-oxo-4,5-dihydro-3-pyrazolyle, 2-oxo-4-pyrrolidinyle, 4-méthyl-5-oxo-4,5-dihydro-1,2,4-triazol-3-yle, ou 5(4)-méthyl-2-oxo-2,3-dihydro-4(5)-imidazolyle et

X représente une valence libre, le groupe vinylène ou le groupe éthylène, caractérisé en ce que de manière connue en soi, on cyclise un composé de formule VI

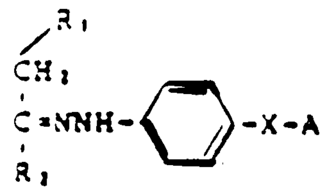

(VI),

dans laquelle $R_1$, $R_2$, A et X ont les significations mentionnées ci-dessus, selon une synthèse d'indole de Fischer, et ensuite, on transforme éventuellement les composés de formule I obtenus en d'autres composés de formule I, ainsi qu'éventuellement, on transforme les composés en leurs sels pharmacologiquement acceptables.

2. Procédé selon la revendication 1 pour la préparation de composés de formule I, dans laquelle
$R_1$ représente l'hydrogène, le groupe méthyle, éthyle, isopropyle ou phényle,
$R_2$ représente le groupe thiophényle, thiazolyle, thiadiazolyle, pyridinyle, pyrazinyle, pyridazinyle ou le groupe phényle, qui peut être substitué par un groupe aminocarbonyle, chloro, trifluorométhyle, méthyle, méthoxy, méthylmercapto, hydroxy ou 1-imidazolyle,
A représente le groupe 3-oxo-2,3,4,5-tétrahydro-6-pyridazinyle, 5-méthyl-3-oxo-2,3,4,5-tétrahydro-6-pyridazinyle, 2-oxo-1,2-dihydro-5-pyrazinyle, 2-oxo-2,3-dihydro-6H-1,3,4-oxadiazine-5-yle, 3-oxo-2,3,4,5-tétrahydro-1,2,4-triazine-6-yle, 5-oxo-4,5-dihydro-6H-1,3,4-oxadiazine-2-yle, 4-méthyl-5-oxo-4,5-dihydro-1,2,4-triazol-3-yle ou 4,4-diméthyl-5-oxo-4,5-dihydro-3-pyrazolyle et
X représente une valence libre ou le groupe vinylène.

3. Procédé selon la revendication 1 ou 2, pour la préparation du 2-(4-pyridyl)-5-(5-méthyl-3-oxo-2,3,4,5-tétrahydro-6-pyridazinyl)-indole et du 2-(4-pyridyl)-5-(3-oxo-2,3,4,5-tétrahydro-6-pyridazinyl)-indole.

4. Procédé pour la préparation de Composés de formule VI

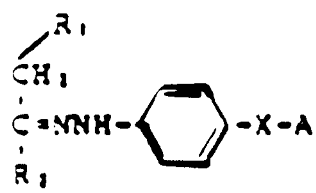

(VI),

dans laquelle $R_1$, $R_2$, X et A ont les significations mentionnées dans la revendication 1, caractérisé en ce que, selon des procédés connus en soi, l'amine de formule III

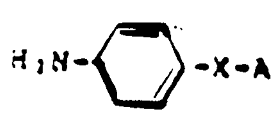

(III),

dans laquelle X et A ont les significations mentionnées dans la revendication 10, est

A) diazotée, et ensuite, soit

a) réduite en hydrazine de formule IV
dans laquelle X et A ont les significations mentionnées ci-dessus et mise à réagir avec des composés de formule V

$R_2COCH_2R_1$ (V),

dans laquelle $R_1$ et $R_2$ ont les significations mentionnées ci-dessus,
soit

b) mise à réagir dans une réaction de Japp-Klingemann avec un composé de formule VII

$R_1$-$CH_2$
$R_2$-CH-Y (VII)

dans laquelle $R_1$ et $R_2$ ont les significations mentionnées ci-dessus et représentent des groupes activateurs,
et ensuite saponifiée,
ou

B) mise à réagir avec un halogénoacétate VIII

Z-$CH_2$-COOEt (VIII),

où Z représente un halogène, puis saponifiée et nitrosée en composé IX

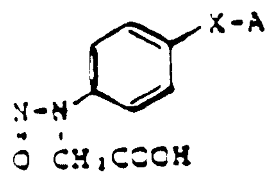

(IX)

dans lequel X et A ont les significations mentionnées, celui-ci est mis à réagir avec des réactifs déshydratants pour donner la sydnone X

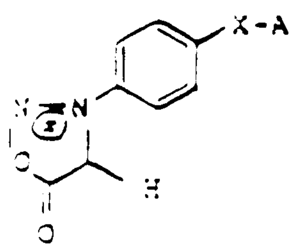

(X)

dans laquelle X et A ont les significations mentionnées, que l'on fait réagir avec les cétones V

$R_2COCH_2R_1$ (V),

dans lesquelles $R_1$ et $R_2$ ont les significations mentionnées ci-dessus, pour donner les composés de formule VI.

**5.** Procédé pour la préparation de médicaments contenant les composés de formule I tels que définis dans la revendication 1, caractérisé en ce que l'on transforme ces composés, conjointement avec des véhicules et/ou adjuvants pharmacologiques, en médicaments.